# EUROPÄISCHE PATENTSCHRIFT

(11) **EP 4 272 270 B1**
(45) Veröffentlichungstag und Bekanntmachung des Hinweises auf die Patenterteilung: **18.03.2026**
(21) Anmeldenummer: 21845079.9
(22) Anmeldetag: 30.12.2021
(51) Int. Cl.: H10K 85/60

(54) **VERBINDUNG UND DEREN VERWENDUNG IN ORGANISCHEN ELEKTRONISCHEN BAUELEMENTEN**
COMPOUND AND ITS USE IN ORGANIC ELECTRONIC DEVICES
COMPOSÉ ET SON UTILISATION DANS DES ÉLÉMENT ÉLECTRONIQUE ORGANIQUES

(30) Priorität: 31.12.2020 DE 102020135172
(43) Veröffentlichungstag der Anmeldung: 08.11.2023
(73) Patentinhaber: Heliatek GmbH, 01139 Dresden (DE)
(72) Erfinder: GERDES, Olga, 89077 Ulm (DE); PFEIFFER, Martin, 01139 Dresden (DE); WEISS, Andre, 01139 Dresden (DE); RAMIREZ, Ivan, 01139 Dresden (DE)
(86) Internationale Anmeldenummer: PCT/EP2021/087875
(87) Internationale Veröffentlichungsnummer: WO 2022/144429

(56) Entgegenhaltungen:
- EP-A1- 3 188 270
- DE-A1- 102013 101 712

## Beschreibung

Die Erfindung betrifft eine Verbindung der allgemeinen Formel(I), eine Verwendung einer solchen Verbindung in einem organischen elektronischen Bauelement, sowie ein organisches elektronisches Bauelement mit einer solchen Verbindung.

Elektronische Bauelemente aus organischen Materialien sind für die Anwendung als LEDs (OLED) und organischen photovoltaischen Elementen (OPV), also organischen Solarzellen, bekannt. Die verwendeten organischen Materialien erfüllen in diesen organischen elektronischen Bauelementen unterschiedliche Funktionen, insbesondere einen Ladungstransport, eine Lichtemission oder Lichtabsorption. Organische Materialien in optoelektronischen Bauelementen können dabei Polymere oder kleine Moleküle sein und in Lösung oder Emulsion durch nasschemische Prozesse wie Coaten oder Drucken oder im Vakuum durch Sublimation zu dünnen Schichten verarbeitet werden. Organisch elektronische Bauelemente können dabei beispielsweise Displays, Datenspeicher oder Transistoren, aber auch organische optoelektronische Bauelemente sein, insbesondere Solarzellen oder Photodetektoren. Solarzellen oder Photodetektoren weisen eine photoaktive Schicht auf, in der bei Einfall von elektromagnetischer Strahlung gebundene Elektronen-Loch-Paare (Exzitonen) als Ladungsträger erzeugt werden. Die Exzitonen gelangen durch Diffusion an eine Grenzfläche, an der Elektronen und Löcher voneinander getrennt werden. Das Material, welches die Elektronen aufnimmt, wird als Akzeptor, und das Material, welches die Löcher aufnimmt, wird als Donor bezeichnet. Weitere organische elektronische Bauelemente sind Licht-emittierende Bauelemente, die Licht aussenden, wenn sie von Strom durchflossen werden. Organische elektronische Bauelemente umfassen mindestens zwei Elektroden, wobei eine Elektrode auf einem Substrat aufgebracht ist und die andere als Gegenelektrode fungiert. Zwischen den Elektroden befindet sich mindestens eine photoaktive Schicht, vorzugsweise eine organische photoaktive Schicht. Weitere Schichten, beispielsweise Transportschichten, können zwischen den Elektroden angeordnet sein.

Es werden nach wie vor organische halbleitende Materialien gesucht, die bei Verwendung in organischen elektronischen Bauelementen zu einer Verbesserung der Eigenschaften der Bauelemente führen. Das Absorptionsspektrum von bekannten Absorbern deckt den blauen Spektralbereich nicht vollständig ab. Zur Erhöhung des Absorptionsbereichs und damit zur Steigerung der Effizienz von Solarzellen werden deshalb Absorber benötigt, die im Spektralbereich zwischen 400 nm und 600 nm, insbesondere zwischen 450 und 550 nm, stark absorbieren und eine Spannung im Bereich von 1 V aufweisen. Dies insbesondere ist für die Verwendung in Tandem- und Triplet-Zellen vorteilhaft Ähnliche Verbindungen wurden bereits in EP 3 188 270 A1 und DE 10 2013 101712 A1 offenbart.

Die Aufgabe wird durch die Gegenstände der unabhängigen Ansprüche gelöst. Vorteilhafte Ausgestaltungen ergeben sich aus den Unteransprüchen.

Die Aufgabe wird insbesondere gelöst durch die Bereitstellung einer Verbindung der allgemeinen Formel I

A1-(T1)ₐ-(T2)_{b}-(Z)_{c}-(T3)_{d}-(T4)ₑ-A2 (I)

mit den Parametern a, b, d, e jeweils unabhängig voneinander 0 oder 1, mit der Maßgabe, dass mindestens einer der Parameter a, b, d, e = 1 ist,
mit dem Parameter c = 1, 2, oder 3,
wobei die allgemeine Gruppe Z ein Block aus zwei Gruppen M und N ist, verknüpft als *-M-N-* oder *-N-M-*, wobei * die Anknüpfung an die Gruppen T1, T2, T3, T4, A1 und A2 bezeichnet,
wobei die Gruppen M jeweils unabhängig voneinander ausgewählt sind aus: 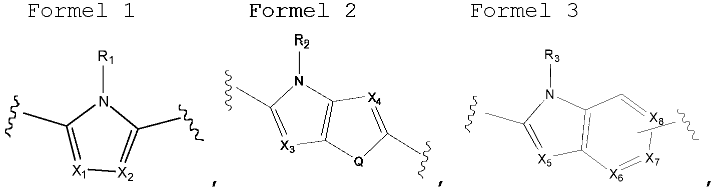
wobei die Gruppen N jeweils unabhängig voneinander ausgewählt sind aus: 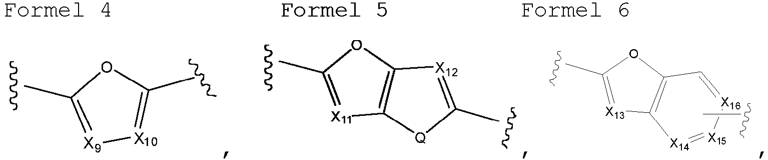
wobei M und N jeweils derart verknüpft sind, dass mindestens ein N-Atom der Gruppe M und ein O-Atom der Gruppe N jeweils über 2 C-Atome miteinander verbunden sind, und 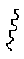 die Anknüpfung an die anderen Gruppen in der Verbindung der allgemeinen Formel I bezeichnet,
mit X₁-X₁₆ unabhängig voneinander ausgewählt aus N oder C-R, mit der Maßgabe, dass in den Gruppen der Formeln 3 und 6 jeweils eine Gruppe aus den Gruppen X₈/X₇ und X₁₆/X₁₅ die Anknüpfung 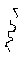 an die anderen Gruppen in der Verbindung der allgemeinen Formel I bezeichnet,
mit R jeweils unabhängig voneinander ausgewählt aus der Gruppe bestehend aus H, Halogen, verzweigtem oder linearem, zyklischem oder offenkettigem C₁-C₁₀-Alkyl, wobei H-Atome des C₁-C₁₀-Alkyls substituiert sein können und C-Atome des C₁-C₁₀-Alkyls durch Heteroatome substituiert sein können, C₂-C₁₀-Alkenyl, O-Alkyl, S-Alkyl, O-Alkenyl, S-Alkenyl, Alkinyl, Aryl, Heteroaryl, wobei bei allen diesen Gruppen H-Atome substituiert sein können, CN, NR₁₀R₁₁, mit R₁₀ und R₁₁ jeweils unabhängig voneinander ausgewählt sind aus H, verzweigtem oder linearem, zyklischem oder offenkettigem C₁-C₁₀-Alkyl, wobei H-Atome des C₁-C₁₀-Alkyls substituiert sein können und C-Atome des C₁-C₁₀-Alkyls durch Heteroatome substituiert sein können,
mit R₁ bis R₃ jeweils unabhängig voneinander ausgewählt aus der Gruppe bestehend aus H, verzweigtem oder linearem, zyklischem oder offenkettigem C₁-C₁₀-Alkyl, wobei H-Atome des C₁-C₁₀-Alkyls substituiert sein können, und C-Atome des C₁-C₁₀-Alkyls durch Heteroatome substituiert sein können, substituiertem oder nicht-substituiertem C₂-C₁₀-Alkenyl, substituiertem oder nicht-substituiertem Aryl, substituiertem oder nicht-substituiertem Heteroaryl, CN,
wobei die elektronenziehenden Gruppen A1 und A2 unabhängig voneinander elektronenziehende Gruppen mit zumindest einer C=C-Doppelbindung sind,
wobei die Gruppen T1, T2, T3 und T4 jeweils unabhängig voneinander ausgewählt sind aus: 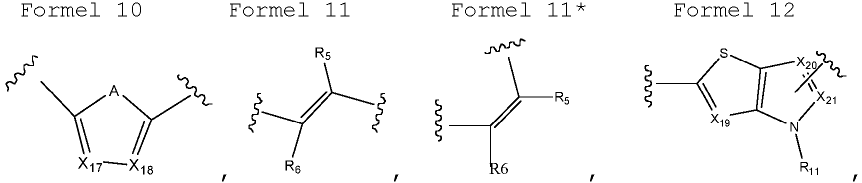 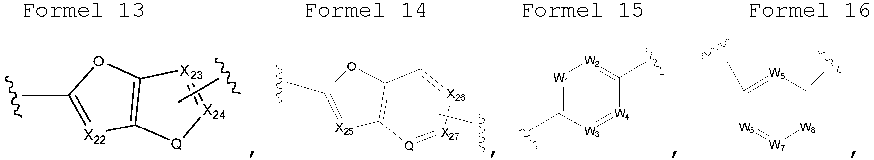
wobei 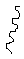 die Anknüpfung an die anderen Gruppen in der Verbindung der allgemeinen Formel I bezeichnet, mit der Maßgabe, dass mindestens eine der Gruppen T1 bis T4 die Formel 10 ist,
mit R₅ und R₆ jeweils unabhängig voneinander ausgewählt aus der Gruppe bestehend aus H, CN, F, Aryl, Heteroaryl, C₂-C₁₀-Alkenyl, Alkinyl, verzweigtem oder linearem, zyklischem oder offenkettigem C₁-C₁₀-Alkyl,
wobei H-Atome des C₁-C₁₀-Alkyls substituiert sein können, wobei, falls der Substituent R₁₃ in der Verbindung der Formel I vorhanden ist, ein Ringschluss zwischen R₅ mit R₁₃ oder R₆ mit R₁₃ möglich ist, mit der Maßgabe, dass sich zwischen R₅ und R₁₃ oder zwischen R₆ und R₁₃ jeweils die Doppelbindung aus Formel 11 befindet, möglich ist,
mit W₁ bis W₈ jeweils unabhängig voneinander ausgewählt aus N, CR, wobei R wie oben beschrieben definiert ist,
mit X₁₇ und X₁₈ unabhängig voneinander ausgewählt aus N und C-R, wobei R wie oben beschrieben definiert ist, mit der Maßgabe, dass zumindest X₁₇ oder X₁₈ N ist,
mit X₁₉ bis X₂₇ unabhängig voneinander ausgewählt aus N und C-R, wobei R wie oben beschrieben definiert ist, und mit der Maßgabe, dass in den Gruppen der Formeln 12, 13 und 14 jeweils eine Gruppe aus den Gruppen X₂₀/X₂₁, X₂₃/X₂₄ und X₂₆/X₂₇ die Anknüpfung 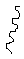 an die anderen Gruppen in der Verbindung der allgemeinen Formel I bezeichnet,
mit A ausgewählt aus der Gruppe bestehend aus S, O, NR₉, und Se,
mit Q ausgewählt aus der Gruppe bestehend aus S, O, NR₉, und Se,
wobei für die Gruppen A und Q der Substituent R₉ jeweils unabhängig voneinander ausgewählt ist aus H, CN, verzweigtem oder linearem, zyklischem oder offenkettigem C₁-C₁₀-Alkyl, wobei die H-Atome des C₁-C₁₀-Alkyl substituiert sein können, C₂-C₁₀-Alkenyl, O-Alkyl, S-Alkyl, O-Alkenyl, S-Alkenyl, Alkinyl, substituiertem oder nicht-substituiertem Aryl, substituiertem oder nicht-substituiertem Heteroaryl.

Das konjugierte π-Elektronensystem des Donorbereichs der erfindungsgemäßen Verbindungen der Formel I kann dadurch über den Donorblock Z hinaus erweitert werden, dass zumindest ein weiterer Donorblock T1, T2, T3 oder T4 eingebaut wird und dementsprechend in der Formel I die zu diesen Donorblöcken dazugehörigen Parameter a, b, d oder e sukzessive auf 1 gesetzt werden.

Möglich ist, dass in der Mittelgruppe Z auch mehrere Zweiergruppen aus M-N bzw. N-M Blöcken aufeinander abfolgen, wenn der Parameter c > 1 ist, z. B. *-M-N-M-N-M-N-*, *-M-N-N-M-M-N-* oder *-N-M-N-M-N-M-N-M-*. Aufgrund dieses strukturellen Merkmales (*-M-N-*)_{c} oder (*-N-M-*)_{c} weisen die vorliegenden Verbindungen eine hohe optische Dichte, bevorzugt im sichtbaren Spektralbereich auf, insbesondere eine hohes Integral über die optische Dichte im Absorptionsspektrum im Vergleich zu nicht erfindungsgemäßen Verbindungen, die das oben beschriebene Strukturelement nicht aufweisen. Unter "Integral" wird dabei der Flächeninhalt unterhalb einer Kurve im Absorptionsspektrum verstanden, das ein wichtiges Merkmal für die Eignung des Materials als organische photosensitives Material ist.

In einer besonders bevorzugten Ausführungsform der Erfindung ist R ausgewählt aus der Gruppe bestehend aus H, Halogen, und einem verzweigten oder linearen, zyklischen oder offenkettigen C₁-C₁₀-Alkyl, wobei bevorzugt H-Atome des C₁-C₁₀-Alkyls teilweise oder vollständig durch F substituiert sind.

In einer bevorzugten Ausführungsform der Erfindung ist mindestens ein H-Atom der Formel 9 substituiert, bevorzugt substituiert durch ein Halogen, bevorzugt F, eine Alkyl-Gruppe, wobei die Alkyl-Gruppe nicht-substituiert oder mindestens ein H-Atom durch ein Halogen, bevorzugt F, substituiert ist, oder eine O-Alkyl-Gruppe.

In einer bevorzugten Ausführungsform der Erfindung ist mindestens ein T1 bis T4 ein substituiertes oder nicht-substituiertes Thiazol und mindestens ein T1 bis T4 ein substituiertes oder nicht-substituiertes Furan oder Thiophen.

In einer bevorzugten Ausführungsform der Erfindung ist die Gruppe M Pyrrol und/oder die Gruppe N Furan, jeweils substituiert oder nicht-substituiert.

Die erfindungsgemäßen chemischen Verbindungen der allgemeinen Formel I weisen Vorteile im Vergleich zum Stand der Technik auf. Vorteilhafterweise können verbesserte Absorber für organische elektronische Bauelemente, insbesondere Solarzellen bereitgestellt werden. Vorteilhafterweise werden Absorbermaterialien für den roten und nah-infraroten Spektralbereich mit einer hohen Absorptionsstärke bereitgestellt. Vorteilhafterweise zeichnen sich die erfindungsgemäßen Verbindungen durch einen in den Blaubereich verschobenen, breiten Absorptionsbereich aus. Vorteilhafterweise zeigen die erfindungsgemäßen Verbindungen ein überraschend gutes Absorptionsverhalten in einem vergleichsweise breiten Bereich des sichtbaren Lichts von 400 nm bis 800 nm, bevorzugt von 400 bis 700 nm, insbesondere eine hohe Absorption im kurzwelligen Spektralbereich von 400 nm bis 600 nm. Vorteilhafterweise ist der Leerlaufspannung Uoc erhöht. Vorteilhafterweise kann die Effizienz von Solarzellen erhöht werden. Vorteilhafterweise sind die erfindungsgemäßen Verbindungen weitgehend rückstandsfrei im Vakuum verdampfbar, und sind damit für eine Vakuumprozessierung zur Herstellung von Solarzellen geeignet. Vorteilhafterweise zeigen die erfindungsgemäßen Verbindungen eine Blauverschiebung im Absorptionsspektrum, insbesondere erfindungsgemäße Verbindungen mit mindestens einem Thiazol der Gruppen T1 bis T4 im Vergleich zu Furan oder Thiophen an dieser Position. Vorteilhafterweise ist die Leerlaufspannung Uoc im Bereich von 1 V oder höher. Dies ist insbesondere vorteilhaft bei Mehrfachzellen, bevorzugt Tandem- oder Triplett-Zellen, oder Mischschichten, da die kleinste Spannung der Schicht limitierend für die Gesamtspannung der Zelle ist. Vorteilhafterweise zeigen erfindungsgemäße Verbindungen im Vergleich zu entsprechenden Verbindungen ohne zumindest ein T1,T2, T3 und T4 mit Formel 10 mit zumindest einem X17 oder X18 einem N eine erhöhte Leerlaufspannung Uoc bei gleichzeitig starker, hinreichend langwelliger Absorption (onset >700nm) und hohem Füllfaktor, bevorzugt >60%, insbesondere bevorzugt >64%, für bulk-heterojunctions mit einem Fulleren, bevorzugt C60.

Die erfindungsgemäßen Verbindungen sind insbesondere sogenannte "kleine Moleküle", wobei darunter nicht-polymere oligomere organische Moleküle mit einer molaren Masse zwischen 100 bis 2000 g/mol verstanden werden, die insbesondere auch monodispers sein können.

In einer bevorzugten Ausführungsform der Erfindung ist T1, T2, T3 oder T4, insbesondere T1 oder T4, ein Thiazol, ein Oxazol, ein Thiadiazol oder ein Oxadiazol.

Gemäß einer Weiterbildung der Erfindung ist vorgesehen, dass die elektronenziehenden Gruppen A1 und A2 unabhängig voneinander ausgewählt sind aus: 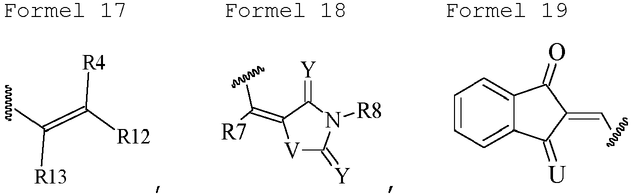
und 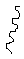 die Anknüpfung an die Gruppen T1 bis T4 und Z in der Verbindung der allgemeinen Formel I bezeichnet,
mit R₄ und R₁₂ jeweils unabhängig voneinander ausgewählt aus H, CN, und COOR, mit der Maßgabe, dass R₄ und R₁₂ nicht beide H sind,
wobei R ausgewählt ist aus der gleichen Gruppe von Verbindungen wie bei R₁ bis R₃ definiert,
mit R₁₃ jeweils unabhängig voneinander ausgewählt aus der Gruppe bestehend aus H, CN, F, Aryl, Heteroaryl, C₂-C₁₀-Alkenyl, Alkinyl, verzweigtem oder linearem, zyklischem oder offenkettigem C₁-C₁₀-Alkyl, wobei H-Atome des C₁-C₁₀-Alkyls substituiert sein können, wobei, falls der Substituent R₅ oder R₆ in der Verbindung der Formel I vorhanden ist, ein Ringschluss zwischen R₅ mit R₁₃ oder R₆ mit R₁₃ möglich ist, mit der Maßgabe, dass sich zwischen R₅ und R₁₃ oder zwischen R₆ und R₁₃ jeweils die Doppelbindung aus Formel 11 befindet,
mit V = O, S; mit Y = O, S, C(CN)₂; mit U = O, S, C(CN)₂,
mit R₇ und R₈ jeweils unabhängig voneinander ausgewählt aus der Gruppe bestehend aus H, CN, F, Aryl, Heteroaryl, C₂-C₁₀-Alkenyl, Alkinyl, verzweigtem oder linearem, zyklischem oder offenkettigem C₁-C₁₀-Alkyl, wobei H-Atome des C₁-C₁₀-Alkyls substituiert sein können, wobei für jede der Gruppen A1 und A2 jeweils für jede C=C-Doppelbindung jeweils unabhängig voneinander sowohl das E-Isomer wie auch das Z-Isomer vorliegen kann.

Für jede C=C-Doppelbindung der Formeln 7, 8 und 9 kann also sowohl das E-Isomer ("E" = entgegen; d.h. trans-Konfiguration) wie auch das Z-Isomer ("Z" = zusammen; d.h. cis-Konfiguration) vorliegen, wobei diese Isomere durch eine gedachte Drehung um 180° um die C=C-Doppelbindungsachse gebildet werden. Dies soll im Folgenden anhand des Beispiels des Restes der Formel 18: 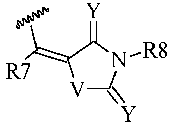 erläutert werden. Beide Isomere, die voneinander getrennt vorhanden sein können, können durch eine gedankliche Drehung um die C=C-Doppelbindung ineinander überführt werden (angedeutet durch den Pfeil an der Doppelbindung), so dass folgende zwei Isomere für die Gruppe der Formel 18 resultieren:

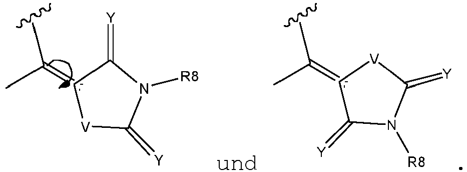

In einer bevorzugten Ausführungsform der Erfindung ist A1 gleich A2.

Bei den erfindungsgemäßen Verbindungen der allgemeinen Formel I können die Aryl-Gruppen und die Heteroaryl- Gruppen bevorzugt C₅-C₁₀-Aryl und C₅-C₁₀-Heteroaryl-Gruppen sein. Als Substituenten werden alle Atome und Atomgruppen außer H verstanden. Als Substituenten kommen insbesondere Halogen, bevorzugt Fluor, aber auch C₁-C₅-Alkylgruppen, die wiederum substituiert sein können, in Betracht. Bei den O-Alkyl, S-Alkyl, O-Alkenyl, S-Alkenyl, Alkinyl-Gruppen kann es sich jeweils um C₁-C₁₀-Gruppen handeln, bevorzugt um C₁-C₅-Gruppen.

Die zyklischen oder offenkettigen C₁-C₁₀-Alkyl-Gruppen der erfindungsgemäßen Verbindungen der Formel I können dabei linear oder auch verzweigt sein und sind bevorzugt C₁-C₅-Alkyl-Gruppen. Nicht benachbarte und nicht endständige C-Atome dieser Alkyl-Gruppen können durch Heteroatome ersetzt sein.

Unter "Heteroatomen" im Sinne der vorliegenden Verbindungen der Formel I werden insbesondere O, S, Se oder NR verstanden, wobei der Substituent R definiert ist, wie die Substituenten R₁ bis R₃, die bereits oben beschrieben wurden.

Die Anbindungsstellen bei den einzelnen Gruppen, die mit 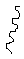 bezeichnet sind, kennzeichnen die Anknüpfungspunkte der jeweiligen Gruppen an die anderen Gruppen der Verbindungen der Formel I, also z. B. bei der elektronenziehenden Gruppe A1 in der Verbindung der Formel I die Anknüpfung entweder an die Donorgruppen T1 (bei a =1), oder T2 (bei a= 0 und b =1), oder an die Donorgruppe Z wenn die Parameter a und b beide 0 sind.

Gemäß einer Weiterbildung der Erfindung ist vorgesehen, dass die elektronenziehenden Gruppen A1 oder A2 die folgende Gruppe 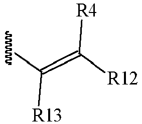 sind, wobei bevorzugt R₄ und R₁₂ CN sind. Derartige elektronenziehende Gruppen A1 und A2 führen zu oligomeren Verbindungen der Formel I, die besonders gut durch Gasphasenabscheidung auf Substrate aufgebracht werden können. Besonders bevorzugt sind R₄ und R₁₂ CN, so dass die besonders stark elektronenziehende Gruppe Dicyano-Vinylen resultiert. Weiterhin kann der Substituent R₁₃ bevorzugt H sein.

Gemäß einer Weiterbildung der Erfindung ist vorgesehen, dass einer der Parameter a, b, d, e = 0 ist, bevorzugt zwei der Parameter a, b, d, e = 0 sind, insbesondere bevorzugt drei der Parameter a, b, d, e = 0 sind, und/oder mindestens einer der Parameter a, b = 1 und/oder einer der Parameter d, e = 1 ist, bevorzugt einer der Parameter a, b = 0 und einer der Parameter d, e = 0 ist, insbesondere bevorzugt a und b = 1 und d und e = 1 sind.

Gemäß einer Weiterbildung der Erfindung ist vorgesehen, dass in der der allgemeinen Formel I der Parameter c = 1 ist

A1-(T1)ₐ-(T2)_{b}-Z-(T3)_{d}-(T4)ₑ-A2 (II).

Die Erfinder haben festgestellt, dass ein Donorblock Z ausreichend ist, um eine erhöhte optische Dichte gegenüber strukturell verschiedenen Verbindungen zu erzielen.

Gemäß einer Weiterbildung der Erfindung ist vorgesehen, dass Z = *-M-N-* oder *-N-M-*und M eine Gruppe der 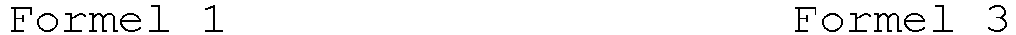 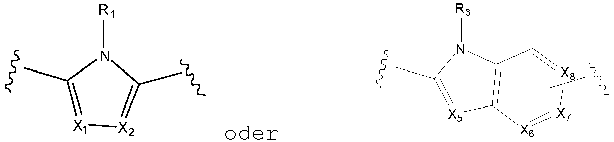 ist, wobei bevorzugt
X₁ und X₂ unabhängig voneinander ausgewählt sind aus C-R mit R jeweils unabhängig voneinander ausgewählt aus der Gruppe bestehend aus H, Halogen, verzweigtem oder linearem, zyklischem oder offenkettigem C₁-C₁₀-Alkyl.

Gemäß einer Weiterbildung der Erfindung ist vorgesehen, dass in der allgemeinen Formel I der Parameter c = 1 ist

A1-(T1)ₐ-(T2)_{b}-Z-(T3)_{d}-(T4)ₑ-A2 (II),

wobei bevorzugt T1 oder T2, und T3 oder T4 unabhängig voneinander Formel 10 sind. Diese einfachen Pyrrol-Struktureinheiten für den Donorblock M, die kein weiteres kondensiertes aromatisches π-Elektronensystem aufweisen, führen bereits zu einer merklichen Zunahme der Absorption von Strahlung für die erfindungsgemäßen Verbindungen, wenn diese weiterhin auch die Donorgruppe N aufweisen.

Die Begriffe "substituiert" bzw. "Substituent" sind im Sinne der vorliegenden Erfindung so auszulegen, dass ein oder mehrere H-Atome gegen eine beliebige andere Atom-Gruppe oder ein anderes Atom ausgetauscht sind. "Substituenten" in diesem Sinne können insbesondere ein Halogen oder ein Pseudohalogen, bevorzugt Fluor oder CN, sowie eine Arylgruppe sein, bevorzugt Phenyl, oder eine Alkylgruppe, bevorzugt eine C₁-C₆-Alkyl-Gruppe.

Die allgemeinen Gruppen und Substituenten in dem Donorblock M der allgemeinen Formel I können folgendermaßen definiert sein:
X₁ und X₂ unabhängig voneinander ausgewählt aus C-R mit R jeweils unabhängig voneinander ausgewählt aus der Gruppe bestehend aus H, Halogen, verzweigtem oder linearem, zyklischem oder offenkettigem C₁-C₁₀-Alkyl.

Diese einfachen Furan-Struktureinheiten für den Donorblock N, die kein weiteres kondensiertes aromatisches π-Elektronensystem aufweisen, führen bereits zu einer merklichen Zunahme der Absorption von Strahlung für die erfindungsgemäßen Verbindungen, wenn diese weiterhin auch die Donorgruppe M aufweisen. Möglich ist aber auch die Verwendung von kondensierten Donorblöcken, die Furan erhalten, wie Benzofurane oder andere unter die allgemeinen Formeln 5 oder 6 fallende Verbindungen. Die allgemeinen Gruppen X₉ und X₁₀ in der Formel 4 können dabei bevorzugt unabhängig C-R sein mit R jeweils unabhängig voneinander ausgewählt aus der Gruppe bestehend aus H, Halogen, verzweigtem oder linearem, zyklischem oder offenkettigem C₁-C₁₀-Alkyl.

Gemäß einer Weiterbildung der Erfindung ist vorgesehen, dass X₁₇ oder X₁₈ = C-R ist, wobei R unabhängig voneinander ausgewählt ist aus einer Gruppe bestehend aus H, Halogen, verzweigtem oder linearem, zyklischem oder offenkettigem C₁-C₁₀-Alkyl, R5 und R6 jeweils unabhängig voneinander ausgewählt aus H, CN, F, Aryl, Heteroaryl, C₂-C₁₀-Alkenyl, Alkinyl, verzweigtem oder linearem, zyklischem oder offenkettigem C₁-C₁₀-Alkyl, wobei H-Atome des C₁-C₁₀-Alkyls substituiert sein können.

Falls der Substituent R₅ und R₆ in der Verbindung vorhanden ist, ist ein Ringschluss zwischen R₅ mit R₁₃ als auch zwischen R₆ mit R₁₃ möglich, mit der Maßgabe, dass sich zwischen R₅ und R₁₃ oder zwischen R₆ und R₁₃ die Doppelbindung aus Formel 11 oder Formel 11* befindet.

Gemäß einer Weiterbildung der Erfindung ist vorgesehen, dass X₁₇ und X₁₈ N sind, und/oder X₁₉ bis X₂₇ unabhängig voneinander ausgewählt sind aus C-R, wobei R wie oben beschrieben definiert ist, und mit der Maßgabe, dass in den Gruppen der Formeln 12, 13 und 14 jeweils eine Gruppe aus den Gruppen X₂₀/X₂₁, X₂₃/X₂₄ und X₂₆/X₂₇ die Anknüpfung 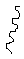 an die anderen Gruppen in der Verbindung der allgemeinen Formel I bezeichnet.

Gemäß einer Weiterbildung der Erfindung ist vorgesehen, dass die Gruppe N die folgende allgemeine Gruppe der Formel 4 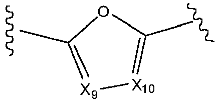 ist,
wobei bevorzugt X₉ und X₁₀ unabhängig voneinander ausgewählt sind aus C-R mit R jeweils unabhängig voneinander ausgewählt aus der Gruppe bestehend aus H, Halogen, verzweigtem oder linearem, zyklischem oder offenkettigem C₁-C₁₀-Alkyl.

Gemäß einer Weiterbildung der Erfindung ist vorgesehen, dass M die allgemeine Gruppe der Formel 3 ist und/oder N die allgemeine Gruppe der Formel 6 ist, wobei bevorzugt in der Formel 10 A = S oder O ist.

Gemäß einer Weiterbildung der Erfindung ist vorgesehen, dass b = 1 und T2 die Gruppe der Formel 10 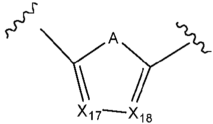 ist,
wobei bevorzugt in der Formel 10 A = S oder O ist, und/oder wobei d = 1 und T3 die Gruppe der Formel 10 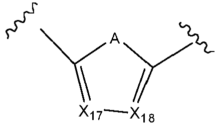 oder der Formel 11 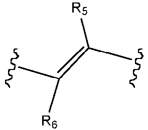 ist,
wobei bevorzugt in der Formel 10 A = S oder O ist.

Gemäß einer Weiterbildung der Erfindung ist vorgesehen, dass e = 1 und T4 die Gruppe der Formel 10 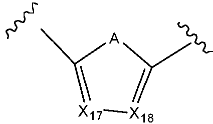 oder der Formel 11 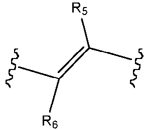 ist,
wobei bevorzugt in der Formel 10 A = O oder S ist, und/oder wobei a = 1 und T1 die Gruppe der Formel 10 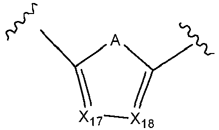 oder der Formel 11 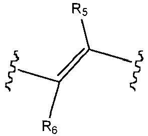 ist,
wobei bevorzugt in der Formel 10 A = S oder O ist.

Gemäß einer Weiterbildung der Erfindung ist vorgesehen, dass die Verbindung (Verbindung 31 ist nicht Teil der beanspruchten Erfindung) ausgewählt ist aus der Gruppe bestehend aus: 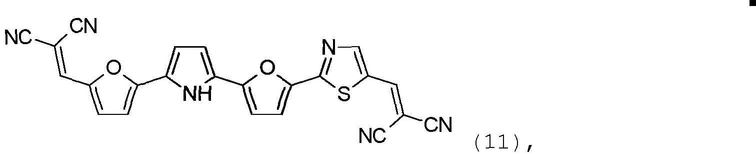 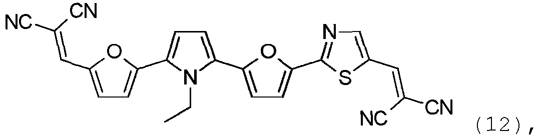 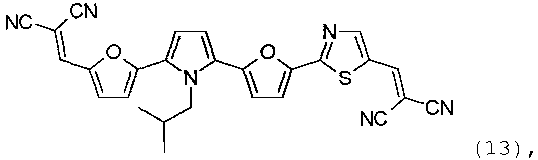 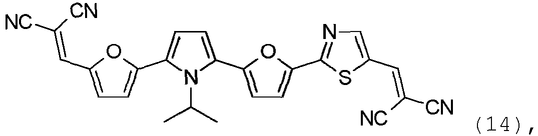 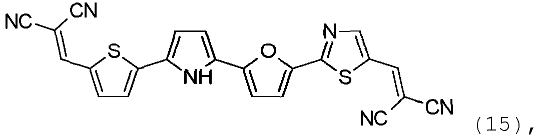 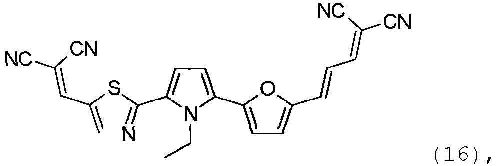 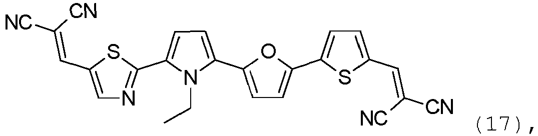 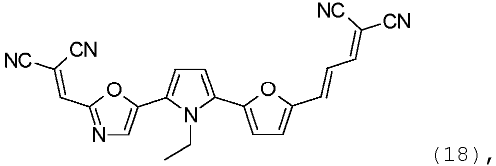 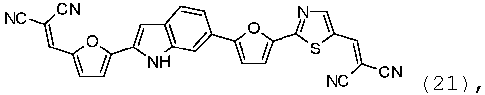 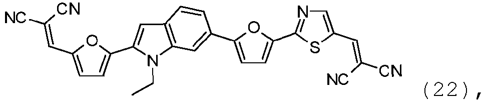 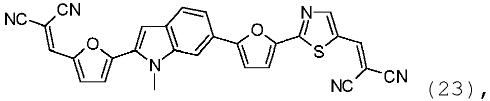 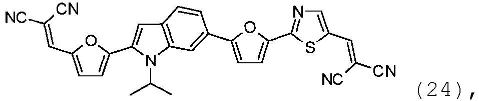 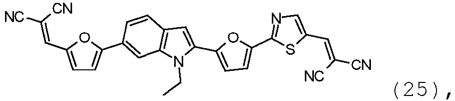 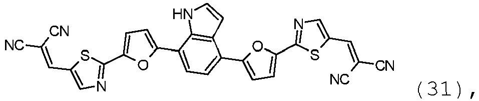 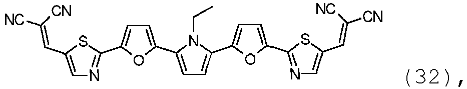 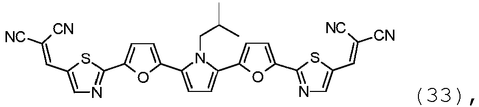 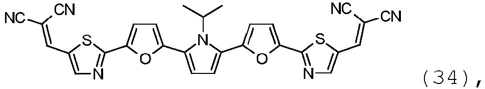 und

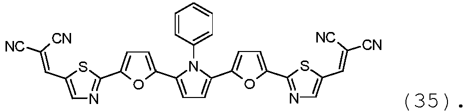

Gemäß einer Weiterbildung der Erfindung ist vorgesehen, dass c = 1, b = 1, und/oder e = 1 ist.

Aufgrund der besonders starken Absorption der erfindungsgemäßen Verbindungen werden besonders gut Exzitonen in Schichten gebildet, die diese Verbindungen umfassen, was bei organischen photoaktiven Bauelementen, die diese Verbindungen umfassen, zu höheren Füllfaktoren FF, verbesserter Leerlaufspannung V_{oc} und verbesserter Kurzschlussstromdichte J_{sc} führt. Bei anderen organischen elektronischen Vorrichtungen sind aufgrund der erhöhten Ladungsträgertransporteigenschaften der erfindungsgemäßen Verbindungen ebenfalls bessere elektronische Werte zu erwarten.

Die Aufgabe der vorliegenden Erfindung wird auch gelöst, indem ein organisches elektronisches Bauelement umfassend mindestens eine erfindungsgemäße Verbindung bereitgestellt wird, insbesondere nach einem der zuvor beschriebenen Ausführungsbeispiele. Dabei ergeben sich für das organische elektronische Bauelement insbesondere die Vorteile, die bereits in Zusammenhang mit der Verbindung der allgemeinen Formel (I) erläutert wurden.

Gemäß einer Weiterbildung der Erfindung ist vorgesehen, dass das organische elektronische Bauelement ein Schichtsystem aufweist, wobei das Schichtsystem eine Elektrode, eine Gegenelektrode und mindestens eine photoaktive Schicht umfasst, wobei die mindestens eine photoaktive Schicht zwischen der Elektrode und der Gegenelektrode angeordnet ist.

Unter einem organischen elektronischen Bauelement wird insbesondere ein elektronisches Bauelement verstanden, welches organische leitende oder halbleitende Materialien aufweist, insbesondere Transistoren, organische lichtemittierende Bauelemente, organische photoaktive Vorrichtungen, bei denen in einer photoaktiven Schicht mittels Bestrahlung Exzitonen (Elektron-Loch-Paare) gebildet werden können, bevorzugt Photodetektoren und Solarzellen.

In einer bevorzugten Ausführungsform der Erfindung weist die mindestens eine photoaktive Schicht mindestens eine erfindungsgemäße Verbindung auf.

Gemäß einer Weiterbildung der Erfindung ist vorgesehen, dass das organische elektronische Bauelement eine organische Solarzelle, ein OFET, eine OLED oder ein organischer Photodetektor ist.

In einer bevorzugten Ausführungsform der Erfindung ist das organische elektronische Bauelement als Tandem- oder Mehrfachzelle ausgebildet, wobei mindestens ein weiteres Absorbermaterial, welches in einem anderen spektralen Bereich des Lichtes absorbiert, vorhanden ist. Als Tandemzelle wird dabei insbesondere eine Solarzelle bezeichnet, die aus einem vertikalen Schichtsystem zweier in Serie verschalteter Zellen besteht. Als Mehrfachsolarzelle wird dabei insbesondere eine Solarzelle bezeichnet, die aus einem vertikalen Schichtsystem mehrerer in Serie verschalteter Zellen besteht.

In einer bevorzugten Ausführungsform der Erfindung ist die erfindungsgemäße Verbindung der allgemeinen Formel (I) ein Absorbermaterial in einer photoaktiven Schicht eines oragnischen elektronischen Bauelements. In einer bevorzugten Ausführungsform der Erfindung ist die erfindungsgemäße Verbindung ein Donor in einem Donor-Akzeptor-Heteroübergang, bevorzugt eingesetzt mit einem Akzeptor ausgewählt aus der Gruppe der Fulleren (C60, C70) bzw. Fullerenderivate, Subphthalocyanine, Rylene, Fluorene, Carbazole, Benzothiadiazole, Diketopyrrolopyrrole und Vinazene.

Die photoaktive Schicht kann dabei eine für die elektronische Funktion des organischen Baudelements wichtige Funktion ausüben, z. B. eine ladungsträgertransportierende Funktion, wie der Transport von Löchern (p-leitend) oder der Transport von Elektronen (n-leitend). Weiterhin kann die photoaktive Schicht auch eine lichtemittierende Schicht umfassen, die bei Anlegen einer Spannung an die Elektrode und Gegenelektrode durch Rekombination der Löcher (positiven Ladungen) und Elektroden (negative Ladung) Strahlung, z. B. Licht emittiert. Bei der organischen Funktionsschicht kann es sich auch um eine photoaktive Schicht handeln in der bei Bestrahlung mit einer Strahlung, z. B. Licht, oder auch UV-Strahlung oder IR-Strahlung Exzitonen (Elektron-Loch-Paare) gebildet werden. Bei organischen photoaktiven Schichten können insbesondere sogenannte flache Heteroübergänge gebildet werden, bei denen eine flache p-leitende Schicht benachbart zu einer flachen n-leitenden Schicht vorhanden ist und die durch Bestrahlung entweder in der p-leitenden oder n-leitenden Schicht gebildeten Exzitonen an der Grenzfläche zwischen beiden Schichten in Löcher und Elektronen getrennt werden können. Weiterhin kann die photoaktive Schicht auch einen sogenannten Volumenheteroübergang umfassen, bei dem p-leitende und n-leitende Materialien in Form eines interpenetrierenden Netzwerks ineinander übergehen, wobei auch dort die Trennung der durch Bestrahlung gebildeten Exzitonen an den Grenzflächen zwischen p-leitenden und n-leitenden Materialien stattfindet.

Exzitonen sind elektrisch neutrale Anregungszustände, die Elektronen-Loch-Paare, die dann in einem weiteren Schritt an einem p-n-Übergang in Elektronen und Löchern getrennt werden. Damit erfolgt die Separation in freie Ladungsträger, die zum elektrischen Stromfluss beitragen. Limitierend ist dabei die Größe der Bandlücke des Halbleiters, entsprechend können nur Photonen absorbiert werden, die eine Energie aufweisen, welche größer als seine Bandlücke ist. Durch das Licht werden immer erst Exzitonen erzeugt, keine freien Ladungsträger, entsprechend ist die rekombinationsarme Diffusion eine für die Höhe des Photostroms wichtige Komponente. Die Exzitonendiffusionslänge muss dabei die typische Eindringtiefe des Lichtes überschreiten, damit ein möglichst großer Teil des Lichtes elektrisch nutzbar ist. Die Exzitonen gelangen durch Diffusion an eine Grenzfläche, wo Elektronen und Löcher voneinander getrennt werden. Das Material, welches die Elektronen aufnimmt, wird als Akzeptor und das Material, welches die Löcher aufnimmt, wird als Donor oder Donator bezeichnet.

Ein bereits literaturbekannter Aufbau einer gängigen organischen Solarzelle besteht in einer pin- oder nip-Dioden [Martin Pfeiffer, "Controlled doping of organic vacuum deposited dye layers: basics and applications", PhD thesis TU-Dresden, 1999 und WO2011/161108A1]: eine pin Solarzelle besteht dabei aus einem Träger/Substrat mit daran anschließendem meist transparentem Grundkontakt, p-Schicht(en), i-Schicht(en), n-Schicht(en) und einem Deckkontakt. Eine nip-Solarzelle besteht aus einem Träger/Substrat mit daran anschließendem meist transparentem Grundkontakt, n-Schicht(en), i-Schicht(en), p-Schicht(en) und einem Deckkontakt. Dabei bedeutet n bzw. p-Dotierung, die zu einer Erhöhung der Dichte freier Elektronen bzw. Löcher im thermischen Gleichgewichtszustand führt. Damit sind solche Schichten primär als Transportschichten zu verstehen. Es ist auch möglich, dass n- oder p-Schicht(en) zumindest teilweise nominell undotiert sind und nur aufgrund der Materialeigenschaften (z.B. unterschiedliche Beweglichkeit) oder aufgrund unterschiedlicher Verunreinigungen (z.B. verbliebene Reste aus der Synthese oder der Schichtherstellung) oder durch Einflüsse der Umgebung (z.B. angrenzende Schichten, Eindiffusion von Metallen oder anderen organischen Materialien, Gasdotierung aus der Umgebungsatmosphäre) bevorzugt n-leitende oder p-leitende Eigenschaften besitzen. In diesem Sinne sind derartige Schichten bevorzugt als Transportschichten zu verstehen. Die Bezeichnung i-Schicht kennzeichnet eine undotierte oder intrinsiche Schicht. Eine oder mehrere i-Schichten können dabei aus einem Material (planar Heterojunctions) als auch aus einer Mischung zweier oder mehrerer Materialien bestehen (bulk heterojunctions), die ein interpenetrierendes Netzwerk aufweisen.

Weiterhin aus der Literatur bekannt sind organische pin-Tandemzellen und pin Mehrfachzellen. WO 2011 161 108 A1 offenbart zur Realisierung ein photoaktives Bauelement mit einer Elektrode und einer Gegenelektrode, wobei zwischen den Elektroden zumindest ein organisches Schichtsystem angeordnet ist, weiterhin mit mindestens zwei photoaktiven Schichtsystemen und zwischen den photoaktiven Schichtsystemen zumindest zwei verschiedene Transportschichtsysteme des gleichen Ladungsträgertyps, dadurch gekennzeichnet, dass ein Transportschichtsystem energetisch an eines der beiden photoaktiven Schichtsysteme angepasst ist und das andere Transportschichtsystem transparent ausgeführt ist.

Die Aufgabe der vorliegenden Erfindung wird auch gelöst, indem eine Verwendung einer erfindungsgemäßen Verbindung in einem organischen elektronischen Bauelement bereitgestellt wird, insbesondere nach einem der zuvor beschriebenen Ausführungsbeispiele. Dabei ergeben sich für die Verwendung der erfindungsgemäßen Verbindung in einem organischen elektronischen Bauelement insbesondere die Vorteile, die bereits in Zusammenhang mit der Verbindung der allgemeinen Formel (I) und dem organischen elektronischen Bauelement mit einer solchen Verbindung erläutert wurden.

In einer bevorzugten Ausführungsform der Erfindung wird die erfindungsgemäße Verbindung, bevorzugt mehrere erfindungsgemäße Verbindungen, in einer Absorberschicht einer Solarzelle verwendet.

Die Erfinder haben festgestellt, dass durch das Vorhandensein insbesondere einer weiteren heterocyclischen Gruppe, die ein Furan- oder ein Thiophenrest sein kann, sowie von Doppelbindungen, die bevorzugt benachbart zu zumindest einer der elektronenziehenden Gruppen A1 und/oder A2 angeordnet sind, aber auch zwischen einer heterocyclischen Gruppe und dem zentralen Donorblock Z angeordnet sein können, weitere erfindungsgemäße Moleküle hergestellt werden können, welche die bereits genannten vorteilhaften Eigenschaften besitzen.

Tabelle 1 zeigt in einer Übersicht die Strukturen, Schmelzpunkte, und Absorptionsmaxima (in nm und eV im Lösungsmittel (LM)) von Ausführungsbeispielen erfindungsgemäßer Verbindungen, die sowohl unter die allgemeinen Formeln I wie II Verbindung 31 ist nicht Teil der beanspruchten Erfindung.

**Tabelle 1:**

| **Nr** | **Struktur** | **Smp./°C^{a}** | **λmax (LM)/nm^{b}** | **in eV** |
|---|---|---|---|---|
| 11 | 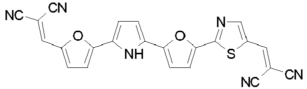 | 288 | 570 | 2,18 |
| 12 | 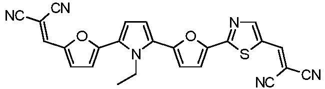 | 289 | 544 | 2,28 |
| 13 | 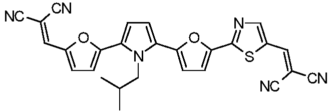 | 270 | 542 | 2,29 |
| 14 | 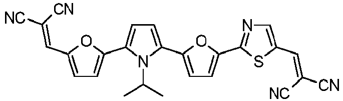 | - ^{c} | 510 | 2,43 |
| 15 | 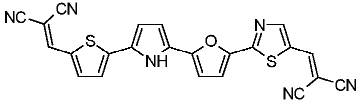 | - ^{c} | 545 | 2,27 |
| 16 | 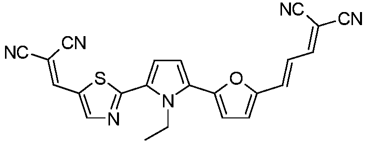 | 256 | 543 | 2,28 |
| 17 | 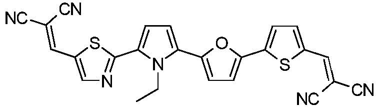 | - ^{d} | - ^{d} | - ^{d} |
| 18 | 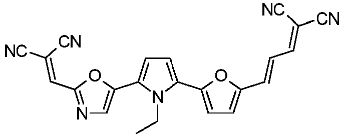 | - ^{d} | - ^{d} | - ^{d} |
| 21 | 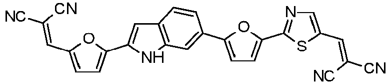 | 307 | 529 | 2,34 |
| 22 | 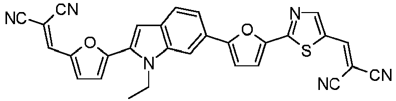 | 352 | 530 | 2,34 |
| 23 | 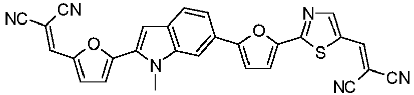 | - ^{d} | - ^{d} | - ^{d} |
| 24 | 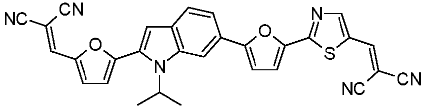 | - ^{d} | - ^{d} | - ^{d} |
| 25 | 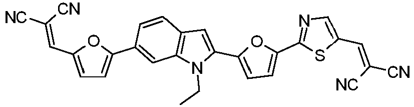 | - ^{d} | - ^{d} | - ^{d} |
| 31 | 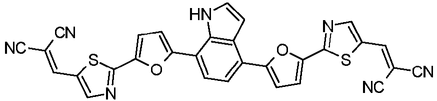 | - ^{c} | 511 | 2,42 |
| 32 | 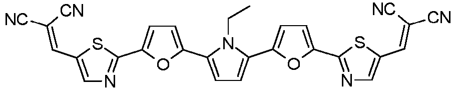 | 320 | 543 | 2,28 |
| 33 | 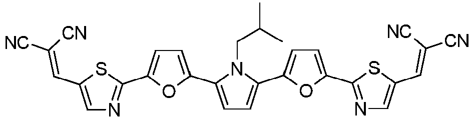 | 291 | 540 | 2,30 |
| 34 | 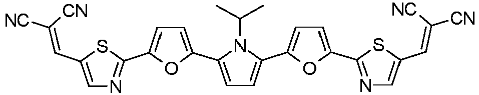 | 332 | 522 | 2,38 |
| 35 | 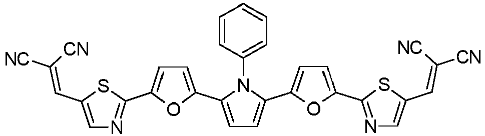 | 323 | 573 | 2,16 |

| | | | | |
|---|---|---|---|---|
| ^{a} onset DSC (dynamische Differenzkalorimetrie; Beginn des Schmelzbereiches; Extrapolierte Anfangstemperatur (Schnittpunkt Wendetangente-Basislinie) ^{b} in Dichlormethan wenn nicht anders vermerkt ^{c} kein Schmelzpeak in DSC ^{d} Daten noch nicht verfügbar | | | | |

Überraschenderweise wurde gefunden, dass die erfindungsgemäßen Verbindungen eine besonders starke Absorption (d.h. hohe optische Dichte am Absorptionsmaximum oder hohes Integral über die optische Dichte im sichtbaren Spektralbereich im Vergleich zu ähnlichen Verbindungen außerhalb des hier beanspruchten Bereichs) aufweisen.

Die folgende Tabelle 2 zeigt verschiedene Parameter erfindungsgemäßer Verbindungen im direkten Vergleich. Die photovoltaischen Parameter V_{oc}, J_{sc} und FF beziehen sich jeweils auf Solarzellen mit 30nm dicker Mischschicht aus dem jeweiligen Donormaterial dieser Verbindungen und Fulleren C60 als photoaktiver Schicht auf Glas mit dem Aufbau

ITO / C60(15 nm) / den jeweiligen Verbindungen:C60 (30nm) /NHT169 oder NHT049 (10nm) / NHT169 oder NHT049:NDP9 (30nm) / NDP9 (1nm) / Au (50nm), gemessen unter AM1.5 Beleuchtung (Am = Air Mass; AM = 1,5 bei diesem Spektrum beträgt die globale Strahlungsleistung 1000 W/m²; AM = 1,5 als Standardwert für die Vermessung von Solarmodulen). Tabelle 2 zeigt insbesondere die optische Dichte am Absorptionsmaximum (ODmax), das optische Integral im sichtbaren Bereich (OD-Integral), sowie V_{oc}, J_{sc}, FF und die Effizienz.

**Tabelle 2**

| **Nr** | **Struktur** | **OD-Integral (400-900nm) [nm]** | **V_{oc} [V]** | **J_{sc} [mA/cm²]** | **FF [%]** | **eff [%]** |
|---|---|---|---|---|---|---|
| 11 | 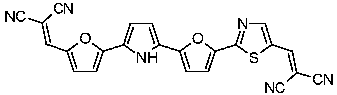 | 88 | 0, 97 | 5,2 | 74,5 | 3,8 |
| 12 | 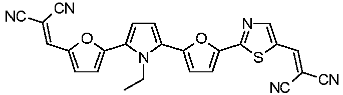 | 72 | 0, 99 | 11,5 | 69,5 | 7, 9 |
| 13 | 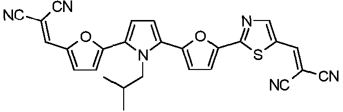 | 110 | 0,98 | 9, 5 | 49,5 | 4, 6 |
| 14 | 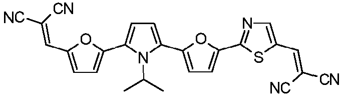 | 37 | - ^{b} | - ^{b} | - ^{b} | - ^{b} |
| 15 | 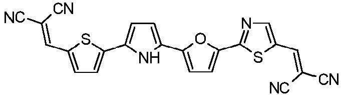 | 92 | - ^{b} | - ^{b} | - ^{b} | - ^{b} |
| 16 | 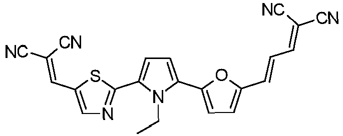 | 107 | 1,02 | 10,7 | 60,7 | 6, 6 |
| 17 | 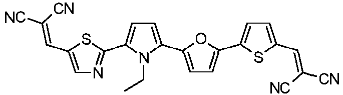 | | | | | |
| 18 | 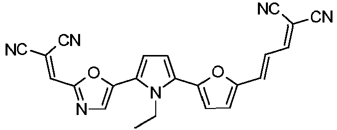 | | | | | |
| 21 | 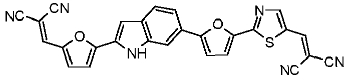 | 98 | - ^{b} | - ^{b} | - ^{b} | - ^{b} |
| 22 | 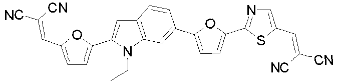 | 79 | 0,99 | 10,5 | 74, 0 | 7,7 |
| 23 | 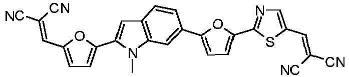 | | | | | |
| 24 | 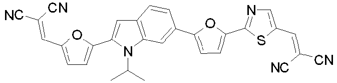 | | | | | |
| 25 | 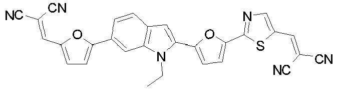 | | | | | |
| 31 | 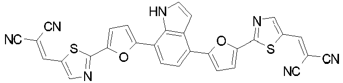 | 55 | - ^{b} | - ^{b} | - ^{b} | - ^{b} |
| 32 | 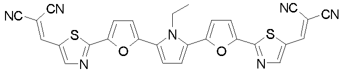 | 87 | 0,94 | 12,8 | 71,2 | 8,6 |
| 33 | 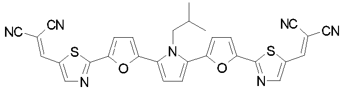 | 85 | 0,95 | 12,4 | 72,4 | 8,5 |
| 34 | 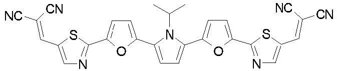 | 97 | - ^{b} | - ^{b} | - ^{b} | - ^{b} |
| 35 | 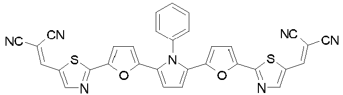 | 101 | 0,97 | 13,3 | 70,6 | 9,1 |

| | | | | | | |
|---|---|---|---|---|---|---|
| ^{b} Zelle nicht gemessen | | | | | | |

Die spektralen Daten beziehen sich auf 30nm dicke Vakuumaufdampfschichten auf Quarzglas.

Es konnte darüber hinaus gezeigt werden, dass viele Derivate der erfindungsgemäßen Verbindungen nicht nur Licht absorbieren sondern auch rückstandsfrei im Vakuum verdampft werden können. Durch sehr gute Ladungstransporteigenschaften und gute Absorptionseigenschaften können hohe Photoströme mit erzeugt werden. Damit können sehr gut kombinierte Tandem-/ Tripel-/ Quadrupel-/ oder Multijunction-Solarzellen hergestellt werden.

Die Erfindung wird im Folgenden anhand der Zeichnungen näher erläutert. Dabei zeigen:
Fig. 1 eine schematische Darstellung eines Ausführungsbeispiels eines organischen elektronischen Bauelements im Querschnitt;
Figur 2 eine Strom-Spannungskurve eines organischen elektronischen Bauelementes mit der Verbindung 12;
Figur 3 eine Strom-Spannungskurve eines organischen elektronischen Bauelementes mit der Verbindung 22; und
Figur 4 eine Strom-Spannungskurve eines organischen elektronischen Bauelementes mit der Verbindung 35.

### Ausführungsbeispiele

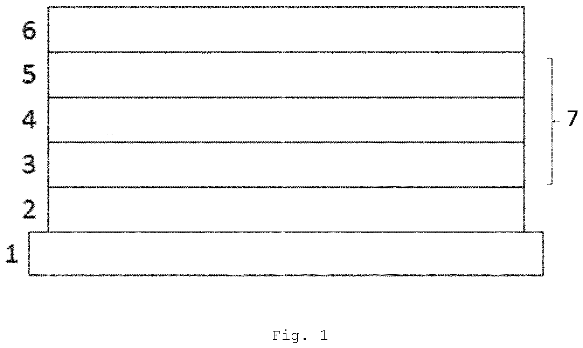

Fig. 1 zeigt eine schematische Darstellung eines Ausführungsbeispiels eines organischen elektronischen Bauelements im Querschnitt;

Das erfindungsgemäße organische elektronische Bauelement weist ein Schichtsystem 7 auf, wobei zumindest eine Schicht des Schichtsystems 7 eine erfindungsgemäße Verbindung der allgemeinen Formel I aufweist.

Das organische elektronische Bauelement umfasst eine erste Elektrode 2, eine zweite Elektrode 6 und ein Schichtsystem 7, wobei das Schichtsystem 7 zwischen der ersten Elektrode 2 und der zweiten Elektrode 6 angeordnet ist. Dabei weist mindestens eine Schicht des Schichtsystems 7 mindestens eine erfindungsgemäße Verbindung der allgemeinen Formel I auf.

Das Schichtsystem 7 weist eine photoaktiven Schicht 4 auf, bevorzugt eine lichtabsorbierende photoaktive Schicht 4, wobei die photoaktive Schicht 4 die mindestens eine erfindungsgemäße Verbindung aufweist.

In einem Ausführungsbeispiel weist die organische Solarzelle ein Substrat 1 auf, z.B. aus Glas, auf dem sich eine Elektrode 2 befindet, die z.B. ITO umfasst. Darauf angeordnet ist das Schichtsystem 7 mit einer elektronentransportierenden Schicht 3 (ETL) sowie einer photoaktiven Schicht 4 mit mindestens einer erfindungsgemäßen Verbindung, einem p-leitenden Donor-Material, und einem n-leitenden Akzeptor-Material, z. B. C60 Fulleren, entweder als flacher Heteroübergang (planar heterojunction) oder als Volumenheteroübergang (bulk heterojunction). Darüber angeordnet befindet sich eine p-dotierte Lochtransportschicht 5 (HTL), und eine Elektrode 6 aus Gold oder Aluminium.

Die Herstellung des Schichtsystems, insbesondere einzelner Schichten, des Bauelements kann durch Verdampfen der Verbindungen im Vakuum, mit oder ohne Trägergas oder prozessieren einer Lösung oder Suspension, wie zum Beispiel beim Coaten oder Drucken geschehen. Einzelne Schichten können ebenso durch Sputtern aufgetragen werden. Vorteilhaft ist die Herstellung der Schichten durch Verdampfen im Vakuum, wobei das Trägersubstrat erwärmt sein kann. Die organischen Materialien werden dabei in Form dünner Filme oder kleiner Volumen auf die Folien aufgedruckt, aufgeklebt, gecoated, aufgedampft oder anderweitig angebracht. Für die Herstellung der dünnen Schichten kommen ebenso alle Verfahren in Betracht, die auch für Elektronik auf Glas, keramischen oder halbleitenden Trägern verwendet werden.

Die chemische Verbindung der allgemeinen Formel I weist folgende Struktur auf:

A1-(T1)ₐ-(T2)_{b}-(Z)_{c}-(T3)_{d}-(T4)ₑ-A2 (I)

mit den Parametern a, b, d, e jeweils unabhängig voneinander 0 oder 1, mit der Maßgabe, dass mindestens einer der Parameter a, b, d, e = 1 ist,
mit dem Parameter c = 1, 2, oder 3,
wobei die allgemeine Gruppe Z ein Block aus zwei Gruppen M und N ist, verknüpft als *-M-N-* oder *-N-M-*, wobei * die Anknüpfung an die Gruppen T1, T2, T3, T4, A1 und A2 bezeichnet,
wobei die Gruppen M jeweils unabhängig voneinander ausgewählt sind aus: 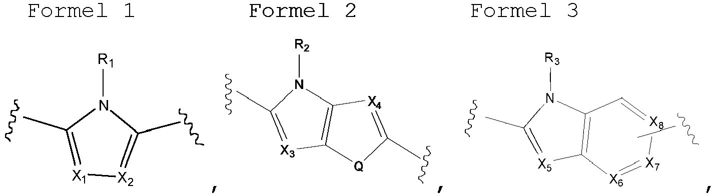
wobei die Gruppen N jeweils unabhängig voneinander ausgewählt sind aus: 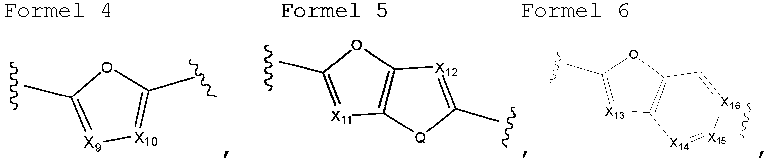
wobei M und N jeweils derart verknüpft sind, dass mindestens ein N-Atom der Gruppe M und ein O-Atom der Gruppe N jeweils über 2 C-Atome miteinander verbunden sind, und 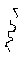 die Anknüpfung an die anderen Gruppen in der Verbindung der allgemeinen Formel I bezeichnet,
mit X₁-X₁₆ unabhängig voneinander ausgewählt aus N oder C-R, mit der Maßgabe, dass in den Gruppen der Formeln 3 und 6 jeweils eine Gruppe aus den Gruppen X₈/X₇ und X₁₆/X₁₅ die Anknüpfung 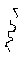 an die anderen Gruppen in der Verbindung der allgemeinen Formel I bezeichnet,
mit R jeweils unabhängig voneinander ausgewählt aus der Gruppe bestehend aus H, Halogen, verzweigtem oder linearem, zyklischem oder offenkettigem C₁-C₁₀-Alkyl, wobei H-Atome des C₁-C₁₀-Alkyls substituiert sein können und C-Atome des C₁-C₁₀-Alkyls durch Heteroatome substituiert sein können, C₂-C₁₀-Alkenyl, O-Alkyl, S-Alkyl, O-Alkenyl, S-Alkenyl, Alkinyl, Aryl, Heteroaryl, wobei bei allen diesen Gruppen H-Atome substituiert sein können, CN, NR₁₀R₁₁, mit R₁₀ und R₁₁ jeweils unabhängig voneinander ausgewählt sind aus H, verzweigtem oder linearem, zyklischem oder offenkettigem C₁-C₁₀-Alkyl, wobei H-Atome des C₁-C₁₀-Alkyls substituiert sein können und C-Atome des C₁-C₁₀-Alkyls durch Heteroatome substituiert sein können,
mit R₁ bis R₃ jeweils unabhängig voneinander ausgewählt aus der Gruppe bestehend aus H, verzweigtem oder linearem, zyklischem oder offenkettigem C₁-C₁₀-Alkyl, wobei H-Atome des C₁-C₁₀-Alkyls substituiert sein können, und C-Atome des C₁-C₁₀-Alkyls durch Heteroatome substituiert sein können, substituiertem oder nicht-substituiertem C₂-C₁₀-Alkenyl, substituiertem oder nicht-substituiertem Aryl, substituiertem oder nicht-substituiertem Heteroaryl, CN,
wobei die elektronenziehenden Gruppen A1 und A2 unabhängig voneinander elektronenziehende Gruppen mit zumindest einer C=C-Doppelbindung sind,
wobei die Gruppen T1, T2, T3 und T4 jeweils unabhängig voneinander ausgewählt sind aus: 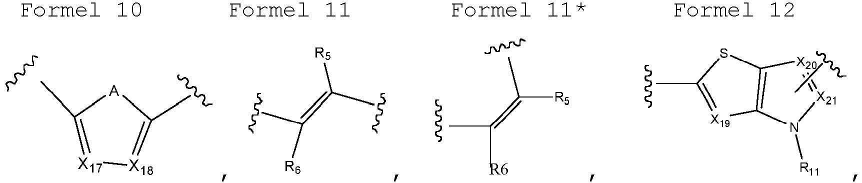 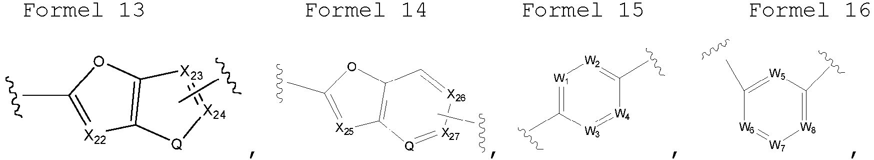
wobei 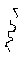 die Anknüpfung an die anderen Gruppen in der Verbindung der allgemeinen Formel I bezeichnet, mit der Maßgabe, dass mindestens eine der Gruppen T1 bis T4 die Formel 10 ist,
mit R₅ und R₆ jeweils unabhängig voneinander ausgewählt aus der Gruppe bestehend aus H, CN, F, Aryl, Heteroaryl, C₂-C₁₀-Alkenyl, Alkinyl, verzweigtem oder linearem, zyklischem oder offenkettigem C₁-C₁₀-Alkyl, wobei H-Atome des C₁-C₁₀-Alkyls substituiert sein können, wobei, falls der Substituent R₁₃ in der Verbindung der Formel I vorhanden ist, ein Ringschluss zwischen R₅ mit R₁₃ oder R₆ mit R₁₃ möglich ist, mit der Maßgabe, dass sich zwischen R₅ und R₁₃ oder zwischen R₆ und R₁₃ jeweils die Doppelbindung aus Formel 11 befindet, möglich ist,
mit W₁ bis W₈ jeweils unabhängig voneinander ausgewählt aus N, CR, wobei R wie oben beschrieben definiert ist,
mit X₁₇ und X₁₈ unabhängig voneinander ausgewählt aus N und C-R, wobei R wie oben beschrieben definiert ist, mit der Maßgabe, dass zumindest X₁₇ oder X₁₈ N ist,
mit X₁₉ bis X₂₇ unabhängig voneinander ausgewählt aus N und C-R, wobei R wie oben beschrieben definiert ist, und mit der Maßgabe, dass in den Gruppen der Formeln 12, 13 und 14 jeweils eine Gruppe aus den Gruppen X₂₀/X₂₁, X₂₃/X₂₄ und X₂₆/X₂₇ die Anknüpfung 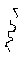 an die anderen Gruppen in der Verbindung der allgemeinen Formel I bezeichnet,
mit A ausgewählt aus der Gruppe bestehend aus S, O, NR₉, und Se,
mit Q ausgewählt aus der Gruppe bestehend aus S, O, NR₉, und Se,
wobei für die Gruppen A und Q der Substituent R₉ jeweils unabhängig voneinander ausgewählt ist aus H, CN, verzweigtem oder linearem, zyklischem oder offenkettigem C₁-C₁₀-Alkyl, wobei die H-Atome des C₁-C₁₀-Alkyl substituiert sein können, C₂-C₁₀-Alkenyl, O-Alkyl, S-Alkyl, O-Alkenyl, S-Alkenyl, Alkinyl, substituiertem oder nicht-substituiertem Aryl, substituiertem oder nicht-substituiertem Heteroaryl.

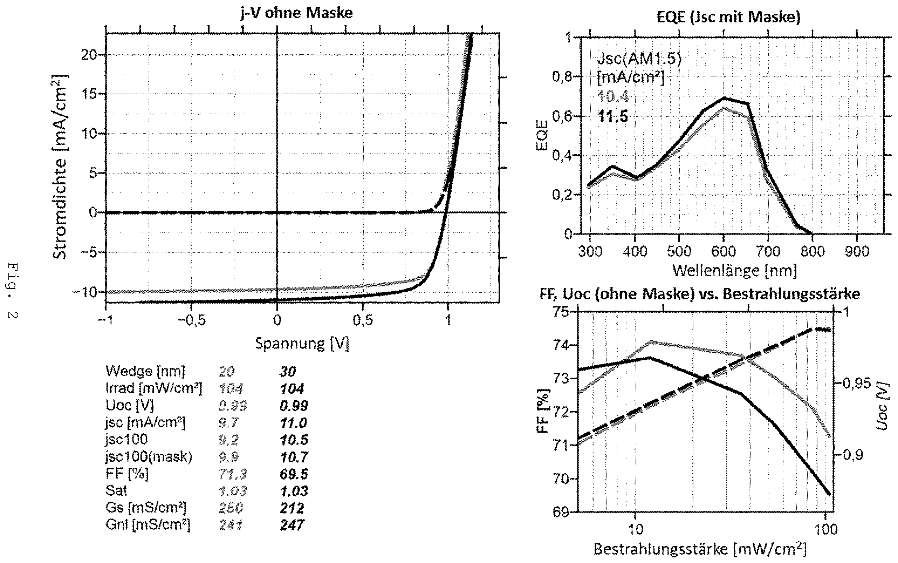

Figur 2 zeigt eine Strom-Spannungskurve eines organischen elektronischen Bauelementes mit der Verbindung 12.

Figur 2 zeigt die Strom-Spannungskurve einer BHJ-Zelle mit dem Aufbau: ITO / C60 (15 nm) / Verbindung 12:C60 (30nm, 2:1, 50 °C) / NHT169 (10nm) / NHT169:NDP9 (30nm, 9,1%wt) / NDP9 (1nm) / Au (50nm), wobei die photoaktive Schicht 4 ein Volumenheteroübergang (bulk heterojunction BHJ) ist. Dabei ist ITO Indiumzinnoxid, NDP9 ein kommerzieller p-Dotand der Novaled GmbH, und NHT169 ein kommerzieller Lochleiter der Novaled GmbH. Das organische elektronische Bauelement ist in diesem Ausführungsbeispiel eine Solarzelle.

In der Solarzelle mit der Verbindung (12) beträgt der Füllfaktor FF 69,5 %, die Leerlaufspannung Uoc 0,99 V und der Kurzschlussstrom Jsc 11,5 mA/cm2. Der Zellwirkungsgrad eines solchen organischen elektronischen Bauelements, insbesondere einer Solarzelle, mit der Verbindung (12) beträgt 7,91%.

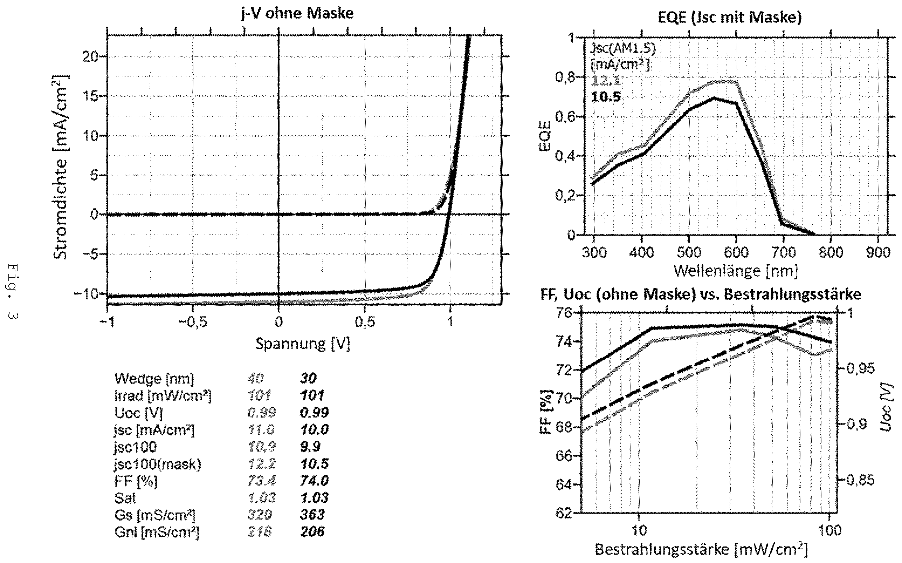

Figur 3 zeigt eine Strom-Spannungskurve eines organischen elektronischen Bauelementes mit der Verbindung 22.

Figur 3 zeigt die Strom-Spannungskurve einer BHJ-Zelle mit dem Aufbau: ITO / C60 (15 nm) / Verbindung 22:C60 (30nm, 3:2, 50 °C) / NHT049 (10nm) / NHT049:NDP9 (30nm, 9,7%wt) / NDP9 (1nm) / Au (50nm), wobei die photoaktive Schicht 4 ein Volumenheteroübergang (bulk heterojunction BHJ) ist. Dabei ist ITO Indiumzinnoxid, NDP9 ein kommerzieller p-Dotand der Novaled GmbH, und NHT049 ein kommerzieller Lochleiter der Novaled GmbH. Das organische elektronische Bauelement ist in diesem Ausführungsbeispiel eine Solarzelle.

In der Solarzelle mit der Verbindung (22) beträgt der Füllfaktor FF 74,0 %, die Leerlaufspannung Uoc 0,99 V und der Kurzschlussstrom Jsc 10,5 mA/cm2. Der Zellwirkungsgrad eines solchen organischen elektronischen Bauelements, insbesondere einer Solarzelle, mit der Verbindung (22) beträgt 7,69%.

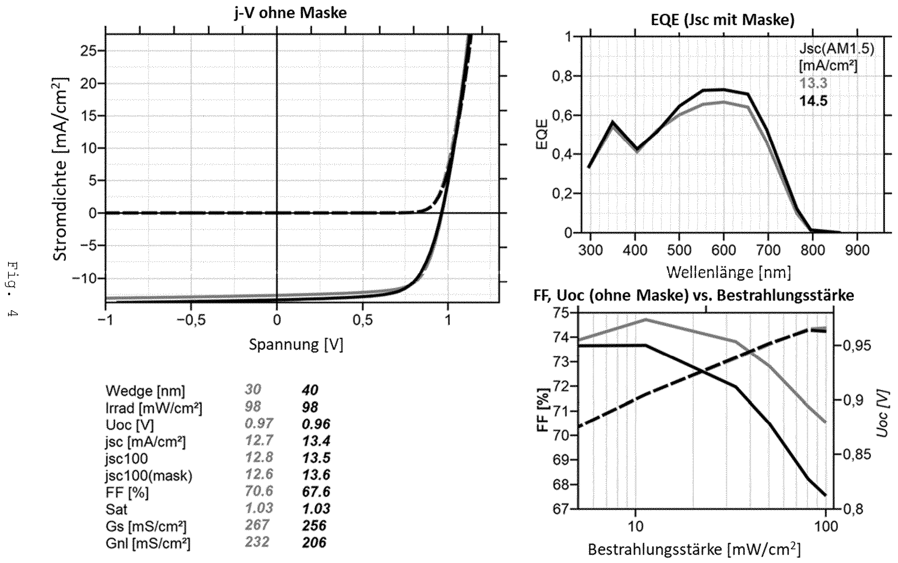

Figur 4 zeigt eine Strom-Spannungskurve eines organischen elektronischen Bauelementes mit der Verbindung 35.

Figur 4 zeigt die Strom-Spannungskurve einer BHJ-Zelle mit dem Aufbau: ITO / C60 (15 nm) / Verbindung 35:C60 (30nm, 2:1, 50 °C) / NHT049 (10nm) / NHT049:NDP9 (30nm, 9,7%wt) / NDP9 (1nm) / Au (50nm), wobei die photoaktive Schicht ein Volumenheteroübergang (bulk heterojunction BHJ) ist. Dabei ist ITO Indiumzinnoxid, NDP9 ein kommerzieller p-Dotand der Novaled GmbH, und NHT049 ein kommerzieller Lochleiter der Novaled GmbH. Das organische elektronische Bauelement ist in diesem Ausführungsbeispiel eine Solarzelle.

In der Solarzelle mit der Verbindung (35) beträgt der Füllfaktor FF 67,6 %, die Leerlaufspannung Uoc 0,96 V und der Kurzschlussstrom Jsc 14,5 mA/cm2. Der Zellwirkungsgrad eines solchen organischen elektronischen Bauelements, insbesondere einer Solarzelle, mit der Verbindung (35) beträgt 9,40%.

Im Folgenden sind exemplarisch Synthesen konkreter Ausführungsbeispiele dargestellt.

Die Synthese der allgemeinen Verbindung (I) kann nach einer der im Folgenden beschriebenen Methoden erfolgen. Diese soll hier als beispielhafte Darstellung dienen und kann in der Reihenfolge ihrer einzelnen Schritte variiert, oder durch andere bekannte Methoden abgewandelt werden. Auch die Zusammenfassung einzelner Reaktionsschritte oder die Veränderung von Teilen der Syntheseroute ist möglich.

### Synthese von {[2-(5-{5-[5-(2,2-dicyanoethenyl)furan-2-yl]-1-ethyl-1H-pyrrol-2-yl}furan-2-yl)-1,3-thiazol-5-yl]methylidene}propanedinitrile (Verbindung 12)

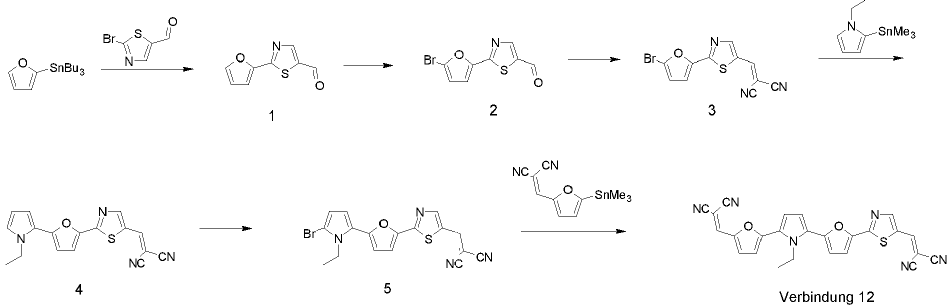

### Synthese von 2-(furan-2-yl)-1,3-thiazole-5-carbaldehyde (1)

4,9 g (25,5 mmol) 2-Bromthiazol-5-carboxaldehyd und 9,4 g (25,5 mmol) 2-(Tributylstannyl)furan wurden in 51 ml Toluol gelöst und das Reaktionsgemisch wurde entgast. 1,55 g (1,28 mmol) Tetrakis-(triphenylphosphin)-palladium(0) wurde zugegeben und das Reaktionsgemisch wurde über Nacht zum Sieden erhitzt. Nachdem das Reaktionsgemisch auf Raumtemperatur abgekühlt war, wurde es mit DCM versetzt. Die organische Phase wurde mit Wasser und ges. NaCl-Lösung gewaschen. Rohprodukt wurde durch Silikagelfiltration in DCM gereinigt, dabei wurde 2,9 g (64% Ausbeute) sauberes **1** isoliert. 1H-NMR in d6-Aceton, ppm: 9,98 (s, 1H), 8,48 (s, 1H), 7,75 (dd, 1H), 7,20 (dd, 1H), 6,63 (dd, 1H).

### Synthese von 2-(5-bromofuran-2-yl)-1,3-thiazole-5-carbaldehyde (2)

7,21 g (40,2 mmol) 1 wurde in 40 ml DMF unter Argon gelöst. 8,03 g (44,2 mmol) NBS wurde in kleinen Portionen dazu gegeben und das Reaktionsgemisch wurde 3 h bei Raumtemperatur gerührt. Das Gemisch wurde auf Eis gegeben. Der ausgefallene Niederschlag wurde filtriert, mit Wasser und Ethanol gewaschen und an der Luft getrocknet. Dabei wurde 9,82 g (95% Ausbeute) **2** isoliert, welches ohne weitere Aufreinigung umgesetzt wurde. 1H-NMR in d6-Aceton, ppm: 10,13 (s, 1H), 8,63 (s, 1H), 7,36 (d, 1H), 6,85 (d, 1H).

### Synthese von {[2-(5-bromofuran-2-yl)-1,3-thiazol-5-yl]methylidene}propanedinitrile (3)

5,24 g (20,3 mmol) 2 wurde in 15 ml Ethanol gelöst. 139 mg (2,03 mmol) β-Alanin und 1,31 g (26,4 mmol) Malodinitril wurden zugegeben. Das Reaktionsgemisch wurde über Nacht bei Raumtemperatur gerührt. Der ausgefallene Niederschlag wurde filtriert, mit Ethanol gewaschen und an der Luft getrocknet. Dabei wurde 5,57 g (90% Ausbeute) **3** isoliert, welches ohne weitere Aufreinigung umgesetzt wurde. 1H-NMR in d6-Aceton, ppm: 8,50 (s, 1H), 8,42 (s, 1H), 7,32 (d, 1H), 6,86 (d, 1H).

### Synthese von ({2-[5-(1-ethyl-1H-pyrrol-2-yl)furan-2-yl]-1,3-thiazol-5-yl}methylidene)propanedinitrile (4)

1,55 g (6 mmol) 1-ethyl-2-(trimethylstannyl)-1H-pyrrole und 2,2 g (7,2 mmol) **3** wurden in 11 ml Dioxan gelöst und die Lösung wurde entgast. 0,15 g (0,3 mmol) Bis-(tri-tert.-butylphosphin)-palladium(0) wurde zugegeben und das Reaktionsgemisch wurde bei 60°C über Nacht gerührt. Nachdem das Reaktionsgemisch auf Raumtemperatur abgekühlt war, wurde es mit DCM versetzt. Die organische Phase wurde mit Wasser gewaschen. Das Rohprodukt wurde umkristallisiert aus Ethanol, dabei wurde 0,63 g **4** isoliert. 1H-NMR in d6-Aceton, ppm: 8,58 (s, 1H), 8,54 (s, 1H), 7,53 (d, 1H), 7,05 (dd, 1H), 6,82 (d, 1H), 6,73 (dd, 1H), 6,19 (dd, 1H), 4,43 (qa, 2H), 1,43 (t, 3H).

### Synthese von ({2-[5-(5-bromo-1-ethyl-1H-pyrrol-2-yl)furan-2-yl]-1,3-thiazol-5-yl}methylidene)propanedinitrile (5)

352 mg (1,1 mmol) **4** wurde in 17 ml DMF gelöst. 186 mg (1,04 mmol) NBS wurde zugegeben und das Reaktionsgemisch wurde über Nacht bei Raumtemperatur gerührt. Anschließend, wurde das Gemisch auf Eis gegeben. Der ausgefallene Niederschlag wurde filtriert, mit Wasser und Ethanol gewaschen und an der Luft getrocknet. Dabei wurde 356 mg (81% Ausbeute) **5** isoliert, welches ohne weitere Aufreinigung umgesetzt wurde. 1H-NMR in d6-Aceton, ppm: 8,59 (s, 1H), 8,56 (s, 1H), 7,53 (d, 1H), 6,91 (d, 1H), 6,75 (d, 1H), 6,34 (d, 1H), 4,38 (qa, 2H), 1,46 (t, 3H).

Synthese von {[2-(5-{5-[5-(2,2-dicyanoethenyl)furan-2-yl]-1-ethyl-1H-pyrrol-2-yl}furan-2-yl)-1,3-thiazol-5-yl]methylidene}propanedinitrile (Verbindung 12) 226 mg (0,57 mmol) 5 und 208 mg (0,68 mmol) 5-(Trimethylstannyl)-2-dicyanovinylfuran wurden gelöst in 1,7 ml Dioxan. Das Reaktionsgemisch wurde entgast und 7,23 mg (0,0143 mmol) Bis-(tri-tert.-butylphosphin)-palladium(0) wurde zugegeben. Das Gemisch wurde bei 80°C über Nacht erhitzt. Das Reaktionsgemisch wurde auf Raumtemperatur abgekühlt und ausgefallene Niederschlag wurde filtriert. Das Rohprodukt wurde mit Methanol gewaschen und aus Acetonitril umkristallisiert. Dabei wurde 60 mg (23% Ausbeute, HPLC-Reinheit 97,5% bei 481 nm) Verbindung 12 isoliert.

### Synthese von {[2-(5-{2-[5-(2,2-dicyanoethenyl)furan-2-yl]-1-ethyl-1H-indol-6-yl}furan-2-yl)-1,3-thiazol-5-yl]methylidene}propanedinitrile (Verbindung 22)

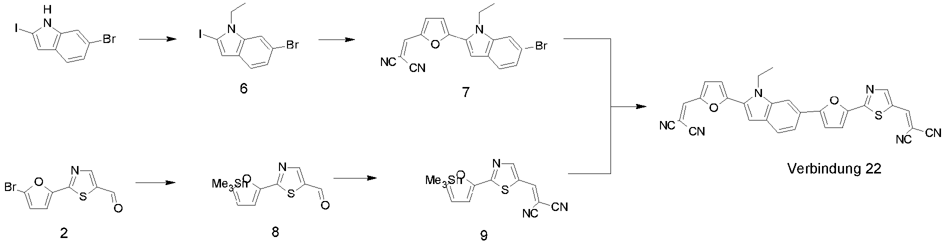

### Synthese von 6-bromo-1-ethyl-2-iodo-1H-indole (6)

5 g (15,5 mmol) 6-bromo-1-ethyl-2-iodo-1H-indole wurde gelöst in 155 ml DMF. 2,59 g (23,3 mmol) Ethylbromid und 4,33 g (31 mmol) Kaliumcarbonat wurden zugegeben und das Gemisch wurde bei Raumtemperatur über Nacht gerührt. Anschließend, wurde das Gemisch mit Wasser versetzt und mit Ethylacetat extrahiert. Organische Phase wurde mit ges. NaCl und mit Wasser gewaschen. Rohprodukt wurde gereinigt durch Silikagelfiltration. Dabei wurde 4,71 g (87% Ausbeute) **6** isoliert. 1H-NMR in d6-Aceton, ppm: 7,69 (d, 1H), 7,42 (d, 1H), 7,11 (dd, 1H), 6,78 (s, 1H), 4,29 (qa, 2H), 1,27 (t, 3H).

### Synthese von {[5-(6-bromo-1-ethyl-1H-indol-2-yl)furan-2-yl]methylidene}propanedinitrile (7)

4,72 g (13,5 mmol) 6 und 4,14 g (13,5 mmol) 5-(Trimethylstannyl)-2-dicyanovinylfuran wurden gelöst in Dioxan und das Gemisch wurde entgast. 0,234 g (0,34 mmol) (3-Chlorpyridyl)-(1,3-diisopropylimidazol-2-yliden)-palladium(II)-dichlorid und 0,256 g (1,69 mmol) Cäsiumfluorid wurden zugeben. Das Reaktionsgemisch wurde gerührt über Nacht bei 80°C. Anschließend, wurde das Gemisch mit DCM versetzt und mit Wasser gewaschen. Das Rohprodukt wurde mit Ethanol versetzt und für 1 h bei Raumtemperatur gerührt. Der Feststoff wurde abfiltriert, mit Ethanol gewaschen und getrocknet. Dabei wurde 1,71 g (35% Ausbeute) **7** isoliert. 1H-NMR in d6-Aceton, ppm: 8,06 (s, 1H), 7,85 (m, 1H), 7,64 (m, 2H), 7,36 (d, 1H), 7,29 (m, 2H), 4,72 (qa, 2H), 1,42 (t, 3H).

### Synthese von 2-[5-(trimethylstannyl)furan-2-yl]-1,3-thiazole-5-carbaldehyde (8)

1,52 g (15,1 mmol) 1-Methylpiperazin wurde in 50 ml trock. THF gelöst und unter Argon auf -78°C abgekühlt. 6 ml (15,1 mmol) 2,5-M-nBuLi wurde langsam zugegeben und das Gemisch wurde gerührt für 15 min bei -78°C. Die Lösung aus 3,54 g (13,7 mmol) **2** in 18 ml trock. THF wurde bei -78°C zugegeben und das Gemisch wurde bei -78°C für weitere 15 min gerührt. 1,91 g (16,4 mmol) TMEDA und 6,58 ml (16,4 mmol) 2,5-M-nBuLi wurden nach einander zugegeben und das Reaktionsgemisch wurde bei -78°C für 2 h gerührt. Anschließend, wurde 16,4 ml (16,4 mmol) 1-M-Me3SnCl-Lösung zugegeben und das Gemisch wurde über Nacht bei Raumtemperatur gerührt. Das Reaktionsgemisch wurde mit ges. NaCl-Lösung versetzt und mit MTBE extrahiert. Organische Phase wurde mit Wasser gewaschen und über Natriumsulfat getrocknet. Das Rohprodukt **8,** 4,3 g (92% Ausbeute) wurde ohne weitere Aufreinigung weiter umgesetzt. 1H-NMR in d6-Aceton, ppm: 10,05 (s, 1H), 8,56 (s, 1H), 7,25 (d, 1H), 6,88 (d, 1H), 0,38 (s, 9H).

### Synthese von ({2-[5-(trimethylstannyl)furan-2-yl]-1,3-thiazol-5-yl}methylidene)propanedinitrile (9)

17 g (49,7 mmol) **8** wurde in 48 ml Ethanol gelöst. 4,27 g (64,6 mmol) Malodinitril und 0,45 g (4,96 mmol) β-Alanin wurden zugegeben. Das Reaktionsgemisch wurde über Nacht bei Raumtemperatur gerührt. Der Niederschlag wurde abfiltriert, mit Ethanol gewaschen und getrocknet. Dabei wurde es 12,5 g (65% Ausbeute) **9** isoliert. 1H-NMR in d6-Aceton, ppm: 8,47 (s, 1H), 8,41 (s, 1H), 7,27 (d, 1H), 6,85 (d, 1H), 0,33 (s, 9H).

### Synthese von {[2-(5-{2-[5-(2,2-dicyanoethenyl)furan-2-yl]-1-ethyl-1H-indol-6-yl}furan-2-yl)-1,3-thiazol-5-yl]methylidene}propanedinitrile (Verbindung 22)

549 mg (1,5 mmol) 7 und 585 mg (1,5 mmol) 9 wurden in 9 ml Dioxan gelöst und die Lösung wurde entgast. 19,2 mg (0,04 mmol) Bis-(tritert.-butylphosphin)-palladium(0) wurde zugegeben und das Reaktionsgemisch wurde über Nacht gerührt bei 60°C. Der Niederschlag wurde abfiltriert, mit Methanol gewaschen und zwei Mal aus Chlorbenzol umkristallisiert. Dabei wurde 222 mg (29% Ausbeute, HPLC-Reinheit 94,5% bei 530 nm) von Verbindung 22 isoliert.

### Synthese von 2,2'-{(1-phenyl-1H-pyrrole-2,5-diyl)bis[(furan-5,2-diyl)-1,3-thiazole-2,5-diylmethanylylidene]}dipropanedinitrile (Verbindung 35)

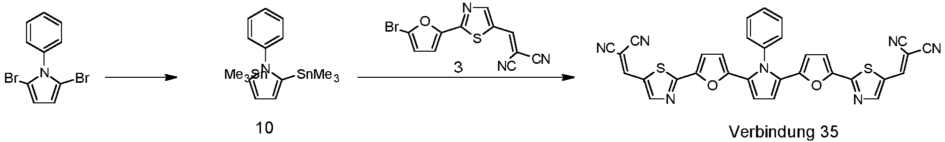

### Synthese von 1-phenyl-2,5-bis(trimethylstannyl)-1H-pyrrole (10)

3,49 g (11,6 mmol) 2,5-dibromo-1-phenyl-1H-pyrrole wurde in 105 ml trock. THF gelöst und die Lösung wurde auf -78°C abgekühlt. 54,6 ml (92,8 mmol) 1,7-M-tBuLi wurde langsam bei -78°C zugetropft und das Gemisch wurde für 3 h bei -78°C gerührt. Anschließend, wurde 92,8 ml (92,8 mmol) 1-M-Me3SnCl-Lösung zugegeben und das Reaktionsgemisch wurde über Nacht bei Raumtemperatur gerührt. Das Reaktionsgemisch wurde mit Petrolether versetzt und mit Wasser extrahiert. Organische Phase wurde über Natriumsulfat getrocknet. Rohprodukt **10**, wurde aus Ethanol umkristallisiert. Dabei wurde 1,48 g (27% Ausbeute) **10** isoliert. 1H-NMR in d6-Aceton, ppm: 7,54 (m, 3H), 7,32 (m, 2H), 6,45 (s, 2H), 0,07 (s, 18H).

### Synthese von 2,2'-{(1-phenyl-1H-pyrrole-2,5-diyl)bis[(furan-5,2-diyl)-1,3-thiazole-2,5-diylmethanylylidene]}dipropanedinitrile (Verbindung 35)

469 mg (1,0 mmol) 10 und 765 mg (2,5 mmol) 3 wurden in 5 ml Dioxan gelöst und die Lösung wurde entgast. 12,8 mg (0,025 mmol) Bis-(tri-tert.-butylphosphin)-palladium(0) wurde zugegeben und ds Reaktionsgemisch wurde bei 80°C über Nacht gerührt. Der Niederschlag wurde abfiltriert, mit Ethanol gewaschen und zwei Mal aus Chlorbenzol umkristallisiert. Dabei wurde 210 mg (35% Ausbeute, HPLC-Reinheit 99,3% bei 573 nm) Verbindung 35 isoliert.

## Patentansprüche

1. Verbindung der allgemeinen Formel I
A1-(T1)ₐ-(T2)_{b}-(Z)_{c}-(T3)_{d}-(T4)ₑ-A2 (I)
- mit den Parametern a, b, d, e jeweils unabhängig voneinander 0 oder 1, mit der Maßgabe, dass mindestens einer der Parameter a, b, d, e = 1 ist,
- mit dem Parameter c = 1, 2, oder 3,
- wobei die allgemeine Gruppe Z ein Block aus zwei Gruppen M und N ist, verknüpft als *-M-N-* oder *-N-M-*, wobei * die Anknüpfung an die Gruppen T1, T2, T3, T4, A1 und A2 bezeichnet,
- wobei die Gruppen M jeweils unabhängig voneinander ausgewählt sind aus: 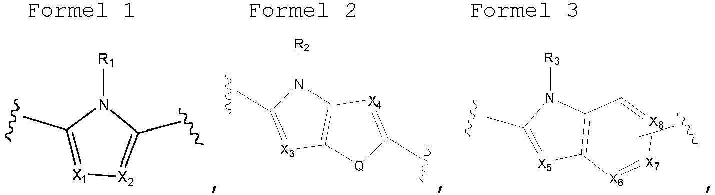
- wobei die Gruppen N jeweils unabhängig voneinander ausgewählt sind aus: 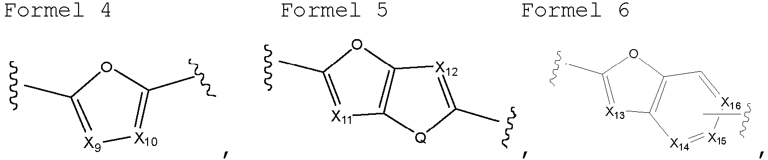
- wobei M und N jeweils derart verknüpft sind, dass mindestens ein N-Atom der Gruppe M und ein O-Atom der Gruppe N jeweils über 2 C-Atome miteinander verbunden sind, und 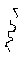 die Anknüpfung an die anderen Gruppen in der Verbindung der allgemeinen Formel I bezeichnet,
- mit X₁-X₁₆ unabhängig voneinander ausgewählt aus N oder C-R, mit der Maßgabe, dass in den Gruppen der Formeln 3 und 6 jeweils eine Gruppe aus den Gruppen X₈/X₇ und X₁₆/X₁₅ die Anknüpfung 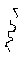 an die anderen Gruppen in der Verbindung der allgemeinen Formel I bezeichnet,
- mit R jeweils unabhängig voneinander ausgewählt aus der Gruppe bestehend aus H, Halogen, verzweigtem oder linearem, zyklischem oder offenkettigem C₁-C₁₀-Alkyl, wobei H-Atome des C₁-C₁₀-Alkyls substituiert sein können und C-Atome des C₁-C₁₀-Alkyls durch Heteroatome substituiert sein können, C₂-C₁₀-Alkenyl, O-Alkyl, S-Alkyl, O-Alkenyl, S-Alkenyl, Alkinyl, Aryl, Heteroaryl, wobei bei allen diesen Gruppen H-Atome substituiert sein können, CN, NR₁₀R₁₁, mit R₁₀ und R₁₁ jeweils unabhängig voneinander ausgewählt sind aus H, verzweigtem oder linearem, zyklischem oder offenkettigem C₁-C₁₀-Alkyl, wobei H-Atome des C₁-C₁₀-Alkyls substituiert sein können und C-Atome des C₁-C₁₀-Alkyls durch Heteroatome substituiert sein können,
- mit R₁ bis R₃ jeweils unabhängig voneinander ausgewählt aus der Gruppe bestehend aus H, verzweigtem oder linearem, zyklischem oder offenkettigem C₁-C₁₀-Alkyl, wobei H-Atome des C₁-C₁₀-Alkyls substituiert sein können, und C-Atome des C₁-C₁₀-Alkyls durch Heteroatome substituiert sein können, substituiertem oder nicht-substituiertem C₂-C₁₀-Alkenyl, substituiertem oder nicht-substituiertem Aryl, substituiertem oder nicht-substituiertem Heteroaryl, CN,
- wobei die elektronenziehenden Gruppen A1 und A2 unabhängig voneinander elektronenziehende Gruppen mit zumindest einer C=C-Doppelbindung sind,
- wobei die Gruppen T1, T2, T3 und T4 jeweils unabhängig voneinander ausgewählt sind aus: 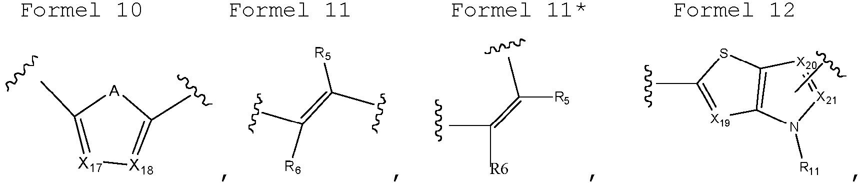 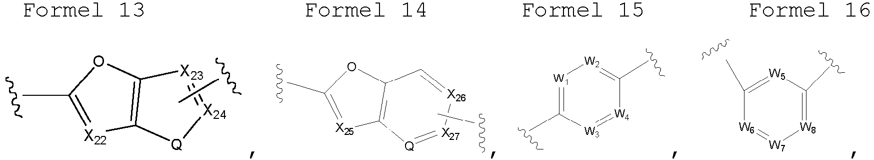
- wobei 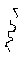 die Anknüpfung an die anderen Gruppen in der Verbindung der allgemeinen Formel I bezeichnet, mit der Maßgabe, dass mindestens eine der Gruppen T1 bis T4 die Formel 10 ist,
- mit R₅ und R₆ jeweils unabhängig voneinander ausgewählt aus der Gruppe bestehend aus H, CN, F, Aryl, Heteroaryl, C₂-C₁₀-Alkenyl, Alkinyl, verzweigtem oder linearem, zyklischem oder offenkettigem C₁-C₁₀-Alkyl, wobei H-Atome des C₁-C₁₀-Alkyls substituiert sein können, wobei, falls der Substituent R₁₃ in der Verbindung der Formel I vorhanden ist, ein Ringschluss zwischen R₅ mit R₁₃ oder R₆ mit R₁₃ möglich ist, mit der Maßgabe, dass sich zwischen R₅ und R₁₃ oder zwischen R₆ und R₁₃ jeweils die Doppelbindung aus Formel 11 befindet, möglich ist,
- mit W₁ bis W₈ jeweils unabhängig voneinander ausgewählt aus N, CR, wobei R wie oben beschrieben definiert ist,
- mit X₁₇ und X₁₈ unabhängig voneinander ausgewählt aus N und C-R, wobei R wie oben beschrieben definiert ist, mit der Maßgabe, dass zumindest X₁₇ oder X₁₈ N ist,
- mit X₁₉ bis X₂₇ unabhängig voneinander ausgewählt aus N und C-R, wobei R wie oben beschrieben definiert ist, und mit der Maßgabe, dass in den Gruppen der Formeln 12, 13 und 14 jeweils eine Gruppe aus den Gruppen X₂₀/X₂₁, X₂₃/X₂₄ und X₂₆/X₂₇ die Anknüpfung 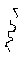 an die anderen Gruppen in der Verbindung der allgemeinen Formel I bezeichnet,
- mit A ausgewählt aus der Gruppe bestehend aus S, O, NR₉, und Se,
- mit Q ausgewählt aus der Gruppe bestehend aus S, O, NR₉, und Se,
- wobei für die Gruppen A und Q der Substituent R₉ jeweils unabhängig voneinander ausgewählt ist aus H, CN, verzweigtem oder linearem, zyklischem oder offenkettigem C₁-C₁₀-Alkyl, wobei die H-Atome des C₁-C₁₀-Alkyl substituiert sein können, C₂-C₁₀-Alkenyl, O-Alkyl, S-Alkyl, O-Alkenyl, S-Alkenyl, Alkinyl, substituiertem oder nicht-substituiertem Aryl, substituiertem oder nicht-substituiertem Heteroaryl.

2. Verbindung nach Anspruch 1, wobei die elektronenziehenden Gruppen A1 und A2 unabhängig voneinander ausgewählt sind aus: 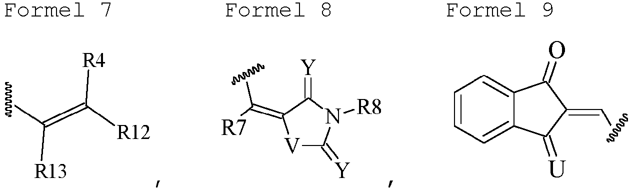 und 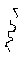 die Anknüpfung an die Gruppen T1 bis T4 und Z in der Verbindung der allgemeinen Formel I bezeichnet,
- mit R₄ und R₁₂ jeweils unabhängig voneinander ausgewählt aus H, CN, und COOR, mit der Maßgabe, dass R₄ und R₁₂ nicht beide H sind,
- wobei R ausgewählt ist aus der gleichen Gruppe von Verbindungen wie bei R₁ bis R₃ definiert,
- mit R₁₃ jeweils unabhängig voneinander ausgewählt aus der Gruppe bestehend aus H, CN, F, Aryl, Heteroaryl, C₂-C₁₀-Alkenyl, Alkinyl, verzweigtem oder linearem, zyklischem oder offenkettigem C₁-C₁₀-Alkyl, wobei H-Atome des C₁-C₁₀-Alkyls substituiert sein können, wobei, falls der Substituent R₅ oder R₆ in der Verbindung der Formel I vorhanden ist, ein Ringschluss zwischen R₅ mit R₁₃ oder R₆ mit R₁₃ möglich ist, mit der Maßgabe, dass sich zwischen R₅ und R₁₃ oder zwischen R₆ und R₁₃ jeweils die Doppelbindung aus Formel 11 befindet,
- mit V = O, S; mit Y = O, S, C(CN)₂; mit U = O, S, C(CN)₂,
- mit R₇ und R₅ jeweils unabhängig voneinander ausgewählt aus der Gruppe bestehend aus H, CN, F, Aryl, Heteroaryl, C₂-C₁₀-Alkenyl, Alkinyl, verzweigtem oder linearem, zyklischem oder offenkettigem C₁-C₁₀-Alkyl, wobei H-Atome des C₁-C₁₀-Alkyls substituiert sein können, wobei für jede der Gruppen A1 und A2 jeweils für jede C=C-Doppelbindung jeweils unabhängig voneinander sowohl das E-Isomer wie auch das Z-Isomer vorliegen kann.

3. Verbindung nach Anspruch 1 oder 2, wobei die elektronenziehenden Gruppen A1 oder A2 die folgende Gruppe 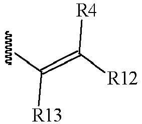 sind, wobei bevorzugt R₄ und R₁₂ CN sind.

4. Verbindung nach einem der vorhergehenden Ansprüche mit einem der Parameter a, b, d, e = 0, bevorzugt mit zwei der Parameter a, b, d, e = 0, insbesondere bevorzugt mit drei der Parameter a, b, d, e = 0, und/oder mit mindestens einem der Parameter a, b = 1 und/oder einem der Parameter d, e = 1, bevorzugt einem der Parameter a, b = 0 und einem der Parameter d, e = 0, insbesondere bevorzugt a und b = 1 und d und e = 1.

5. Verbindung nach einem der vorhergehenden Ansprüche mit c = 1 der allgemeinen Formel II
A1-(T1)ₐ-(T2)_{b}-Z-(T3)_{d}-(T4)ₑ-A2 (II)
mit Z = *-M-N-* oder *-N-M-*und M eine Gruppe der 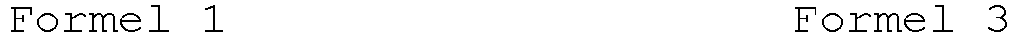 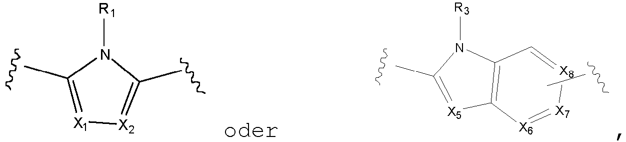 wobei bevorzugt X₁ und X₂ unabhängig voneinander ausgewählt sind aus C-R mit R jeweils unabhängig voneinander ausgewählt aus der Gruppe bestehend aus H, Halogen, verzweigtem oder linearem, zyklischem oder offenkettigem C₁-C₁₀-Alkyl.

6. Verbindung nach einem der vorhergehenden Ansprüche mit c = 1 mit der allgemeinen Formel II
A1-(T1)ₐ-(T2)_{b}-Z-(T3)_{d}-(T4)ₑ-A2 (II),
wobei bevorzugt T1 oder T2, und T3 oder T4 unabhängig voneinander Formel 10 sind.

7. Verbindung nach einem der vorhergehenden Ansprüche, wobei X₁₇ oder X₁₈ = C-R ist, wobei R unabhängig voneinander ausgewählt ist aus einer Gruppe bestehend aus H, Halogen, verzweigtem oder linearem, zyklischem oder offenkettigem C₁-C₁₀-Alkyl, R5 und R6 jeweils unabhängig voneinander ausgewählt sind aus H, CN, F, Aryl, Heteroaryl, C₂-C₁₀-Alkenyl, Alkinyl, verzweigtem oder linearem, zyklischem oder offenkettigem C₁-C₁₀-Alkyl, wobei H-Atome des C₁-C₁₀-Alkyls substituiert sein können.

8. Verbindung nach einem der vorhergehenden Ansprüche mit X₁₇ und X₁₈ N, und/oder mit X₁₉ bis X₂₇ unabhängig voneinander ausgewählt aus C-R, wobei R wie oben beschrieben definiert ist, und mit der Maßgabe, dass in den Gruppen der Formeln 12, 13 und 14 jeweils eine Gruppe aus den Gruppen X₂₀/X₂₁, X₂₃/X₂₄ und X₂₆/X₂₇ die Anknüpfung 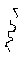 an die anderen Gruppen in der Verbindung der allgemeinen Formel I bezeichnet.

9. Verbindung nach einem der vorhergehenden Ansprüche, wobei die Gruppe N die folgende allgemeine Gruppe der Formel 4 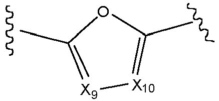 ist, wobei bevorzugt X₉ und X₁₀ unabhängig voneinander ausgewählt sind aus C-R mit R jeweils unabhängig voneinander ausgewählt aus der Gruppe bestehend aus H, Halogen, verzweigtem oder linearem, zyklischem oder offenkettigem C₁-C₁₀-Alkyl.

10. Verbindung nach einem der vorhergehenden Ansprüche, wobei M die allgemeine Gruppe der Formel 3 ist und/oder N die allgemeine Gruppe der Formel 6 ist, wobei bevorzugt in der Formel 10 A = S oder O ist.

11. Verbindung nach einem der vorhergehenden Ansprüche, wobei b = 1 und T2 die Gruppe der Formel 10 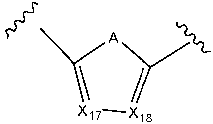 ist, wobei bevorzugt in der Formel 10 A = S oder O ist, und/oder wobei d = 1 und T3 die Gruppe der Formel 10 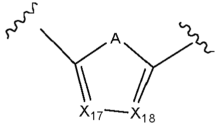 oder der 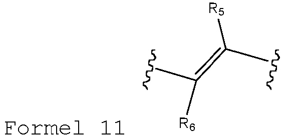 ist, wobei bevorzugt in der Formel 10 A = S oder O ist.

12. Verbindung nach einem der vorhergehenden Ansprüche, wobei e = 1 und T4 die Gruppe der Formel 10 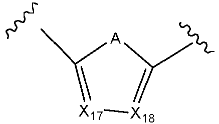 oder der 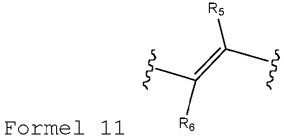 ist, wobei bevorzugt in der Formel 10 A = O oder S ist, und/oder wobei a = 1 und T1 die Gruppe der Formel 10 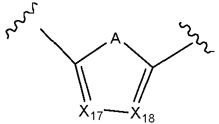 oder der 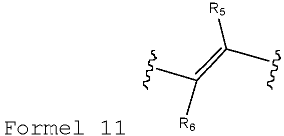 ist, wobei bevorzugt in der Formel 10 A = S oder O ist.

13. Verbindung nach einem der vorhergehenden Ansprüche, wobei die Verbindung ausgewählt ist aus der Gruppe bestehend aus: 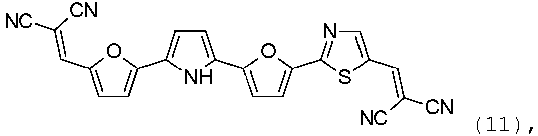 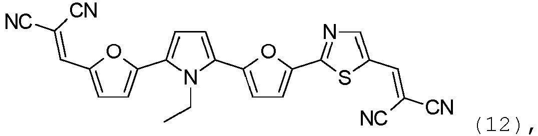 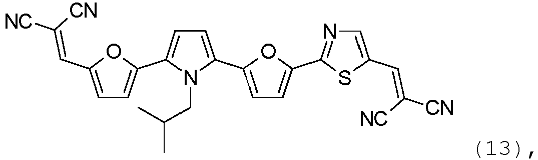 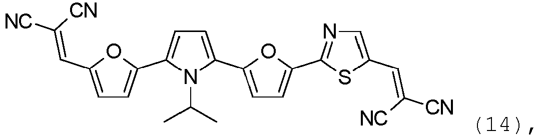 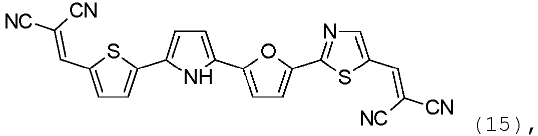 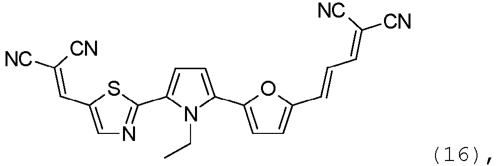 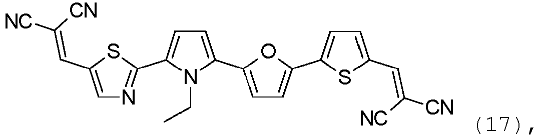 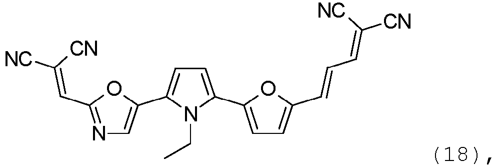 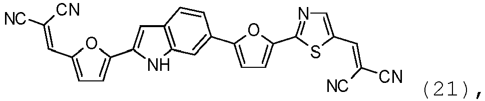 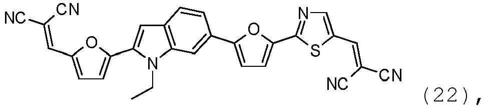 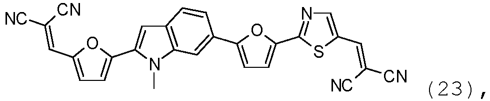 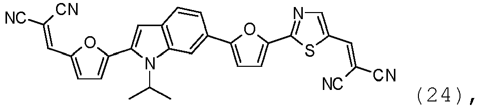 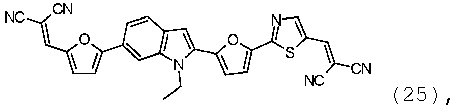 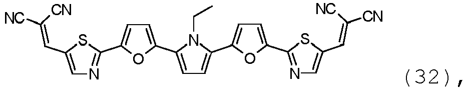 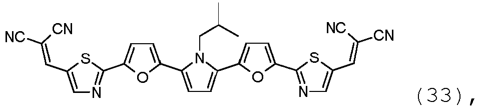 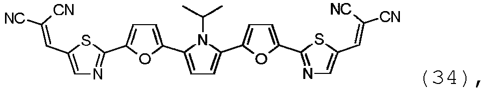 und

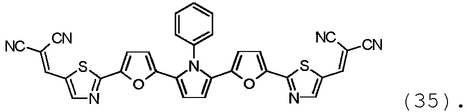

14. Verbindung nach einem der vorhergehenden Ansprüche, wobei c = 1, b = 1, und/oder e = 1 ist.

15. Verwendung mindestens einer Verbindung nach einem der Ansprüche 1 bis 14 in einem organischen elektronischen Bauelement, bevorzugt in einer organischen Solarzelle.

16. Organisches elektronisches Bauelement umfassend mindestens eine Verbindung nach einem der Ansprüche 1 bis 14, bevorzugt umfassend eine Elektrode (2) und eine Gegenelektrode (6) und mindestens eine organische photoaktive Schicht (4) zwischen der Elektrode und der Gegenelektrode, wobei die organische photoaktive Schicht zumindest eine Verbindung nach einem der Ansprüche 1 bis 14 aufweist.

## Claims

1. Compound of general formula I
A1-(T1)ₐ-(T2)_{b}-(Z)_{c}-(T3)_{d}-(T4)ₑ-A2 (I)
- where the parameters a, b, d, e are each independently of one another 0 or 1, with the proviso that at least one of parameters a, b, d, e is equal to 1,
- where the parameter c is equal to 1, 2 or 3,
- wherein the general group Z is a block of two groups M and N linked as *-M-N-* or *-N-M-*, where * denotes the linkage to the groups T1, T2, T3, T4, A1 and A2,
- wherein groups M are each independently of one another selected from: 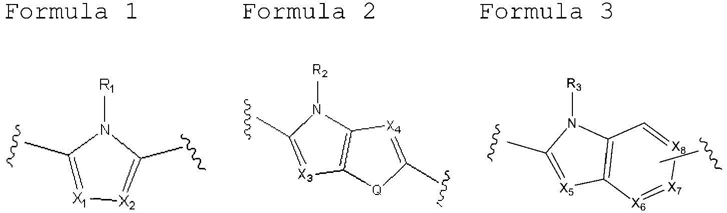
- wherein groups N are each independently of one another selected from: 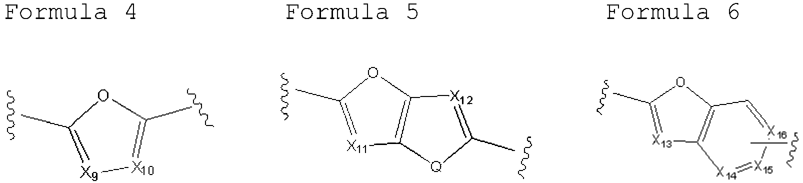
- wherein M and N are each connected such that at least one nitrogen atom of group M and one oxygen atom of group N are in each case connected to one another via 2 carbon atoms, and 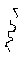 denotes the linkage to the other groups in the compound of general formula I,
- where X₁-X₁₆ are independently of one another selected from N or C-R, with the proviso that, in the groups of formulas 3 and 6, in each case one group from the groups X₈/X₇ and X₁₆/X₁₅ denotes the linkage 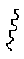 to the other groups in the compound of general formula I,
- where R is in each case independently of one another selected from the group consisting of H, halogen and branched or linear, cyclic or open-chain C₁-C₁₀ alkyl, wherein hydrogen atoms of the C₁-C₁₀ alkyl may be substituted and carbon atoms of the C₁-C₁₀ alkyl may be substituted by heteroatoms, C₂-C₁₀ alkenyl, O-alkyl, S-alkyl, O-alkenyl, S-alkenyl, alkynyl, aryl or heteroaryl, wherein hydrogen atoms in all of these groups may be substituted, CN, NR₁₀R₁₁, where R₁₀ and R₁₁ are each independently of one another selected from H and branched or linear, cyclic or open-chain C₁-C₁₀ alkyl, wherein hydrogen atoms of the C₁-C₁₀ alkyl may be substituted and carbon atoms of the C₁-C₁₀ alkyl may be substituted by heteroatoms,
- where R₁ to R₃ are each independently of one another selected from the group consisting of H and branched or linear, cyclic or open-chain C₁-C₁₀ alkyl, wherein hydrogen atoms of the C₁-C₁₀ alkyl may be substituted, and carbon atoms of the C₁-C₁₀ alkyl may be substituted by heteroatoms, substituted or unsubstituted C₂-C₁₀ alkenyl, substituted or unsubstituted aryl, substituted or unsubstituted heteroaryl or CN,
- wherein the electron-withdrawing groups A1 and A2 are independently of one another electron-withdrawing groups having at least one C=C double bond,
- wherein the groups T1, T2, T3 and T4 are each independently of one another selected from: 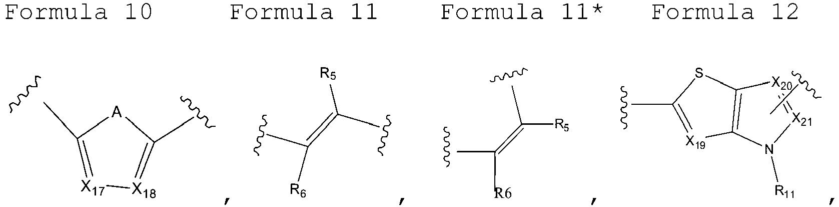 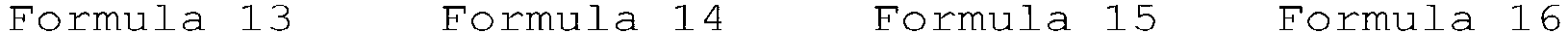 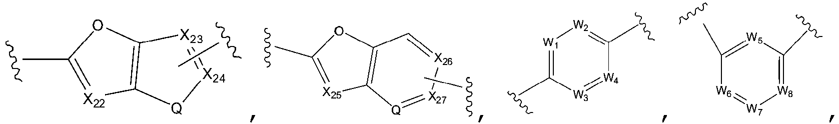
- wherein 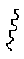 denotes the linkage to the other groups in the compound of general formula I, with the proviso that at least one of groups T1 to T4 is formula 10,
- where R₅ and R₆ are each independently of one another selected from the group consisting of H, CN, F, aryl, heteroaryl, C₂-C₁₀ alkenyl, alkynyl and branched or linear, cyclic or open-chain C₁-C₁₀ alkyl, wherein hydrogen atoms of the C₁-C₁₀ alkyl may be substituted, wherein, if the substituent R₁₃ is present in the compound of formula I, a ring closure between R₅ with R₁₃ or R₆ with R₁₃ is possible, with the proviso that the double bond in formula 11 is in each case possible between R₅ and R₁₃ or between R₆ and R₁₃,
- where W₁ to W₈ are each independently of one another selected from N and CR, wherein R is defined as described above,
- where X₁₇ and X₁₈ are independently of one another selected from N and C-R, wherein R is defined as described above, with the proviso that at least X₁₇ or X₁₆ is N,
- where X₁₉ to X₂₇ are independently of one another selected from N and C-R, wherein R is defined as described above, and with the proviso that in the groups of formulas 12, 13 and 14 in each case one group from the groups X₂₀/X₂₁, X₂₃/X₂₄ and X₂₆/X₂₇ denotes the linkage 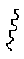 to the other groups in the compound of general formula I,
- where A is selected from the group consisting of S, O, NR₉ and Se,
- where Q is selected from the group consisting of S, O, NR₉ and Se,
- wherein, for groups A and Q, the substituent R₉ is in each case independently of one another selected from H, CN and branched or linear, cyclic or open-chain C₁-C₁₀ alkyl, where the hydrogen atoms of the C₁-C₁₀ alkyl may be substituted, C₂-C₁₀ alkenyl, O-alkyl, S-alkyl, O-alkenyl, S-alkenyl, alkynyl, substituted or unsubstituted aryl, or substituted or unsubstituted heteroaryl.

2. Compound according to Claim 1, wherein the electron-withdrawing groups A1 and A2 are independently of one another selected from: 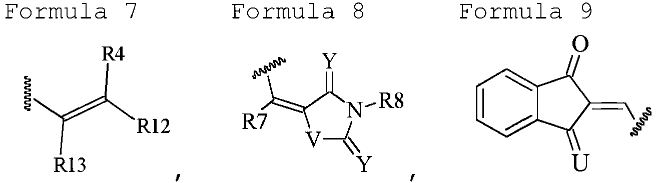 and 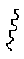 denotes the linkage to groups T1 to T4 and Z in the compound of general formula I,
- where R₄ and R₁₂ are each independently of one another selected from H, CN and COOR, with the proviso that R₄ and R₁₂ are not both H,
- wherein R is selected from the same group of compounds as defined for R₁ to R₃,
- where R₁₃ are each independently of one another selected from the group consisting of H, CN, F, aryl, heteroaryl, C₂-C₁₀ alkenyl, alkynyl and branched or linear, cyclic or open-chain C₁-C₁₀ alkyl, wherein hydrogen atoms of the C₁-C₁₀ alkyl may be substituted, wherein, if the substituent R₅ or R₆ is present in the compound of formula I, a ring closure between R₅ with R₁₃ or R₆ with R₁₃ is possible, with the proviso that the double bond in formula 11 is in each case present between R₅ and R₁₃ or between R₆ and R₁₃,
- where V = O or S; where Y = O, S or C(CN)₂; where U = O, S or C(CN)₂,
- where R₇ and R₈ are each independently of one another selected from the group consisting of H, CN, F, aryl, heteroaryl, C₂-C₁₀ alkenyl, alkynyl and branched or linear, cyclic or open-chain C₁-C₁₀ alkyl, wherein hydrogen atoms of the C₁-C₁₀ alkyl may be substituted, wherein for each of groups A1 and A2 it is in each case possible for each C=C double bond to be present in each case independently of one another as either the E isomer or the Z isomer.

3. Compound according to Claim 1 or 2, wherein the electron-withdrawing groups A1 or A2 are the following group 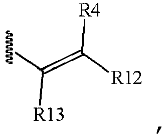 wherein R₄ and R₁₂ are preferably CN.

4. Compound according to any of the preceding claims, where one of parameters a, b, d, e is equal to 0, preferably where two of parameters a, b, d, e is equal to 0, more preferably where three of parameters a, b, d, e is equal to 0, and/or where at least one of parameters a, b is equal to 1 and/or one of parameters d, e is equal to 1, preferably one of parameters a, b is equal to 0 and one of parameters d, e is equal to 0, more preferably a and b are equal to 1 and d and e are equal to 1.

5. Compound according to any of the preceding claims where c = 1 of general formula II
A1-(T1)ₐ-(T2)_{b}-Z-(T3)_{d}-(T4)ₑ-A2 (II)
where Z = *-M-N-* or *-N-M-* and M is a group of 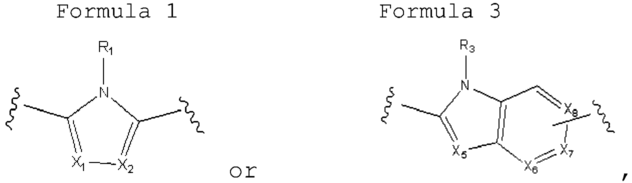 wherein preferably X₁ and X₂ are independently of one another selected from C-R where R is in each case independently of one another selected from the group consisting of H, halogen and branched or linear, cyclic or open-chain C₁-C₁₀ alkyl.

6. Compound according to any of the preceding claims where c = 1 having the general formula II
A1-(T1)ₐ-(T2)_{b}-Z-(T3)_{d}-(T4)ₑ-A2 (II),
wherein preferably T1 or T2, and T3 or T4 are independently of one another formula 10.

7. Compound according to any of the preceding claims, wherein X₁₇ or X₁₈ = C-R, wherein R is independently of one another selected from a group consisting of H, halogen and branched or linear, cyclic or open-chain C₁-C₁₀ alkyl, R5 and R6 are each independently of one another selected from H, CN, F, aryl, heteroaryl, C₂-C₁₀ alkenyl, alkynyl and branched or linear, cyclic or open-chain C₁-C₁₀ alkyl, wherein hydrogen atoms of the C₁-C₁₀ alkyl may be substituted.

8. Compound according to any of the preceding claims where X₁₇ and X₁₈ are N, and/or where X₁₉ to X₂₇ are independently of one another selected from C-R, wherein R is defined as described above, and with the proviso that in the groups of formulas 12, 13 and 14 in each case one group from the groups X₂₀/X₂₁, X₂₃/X₂₄ and X₂₆/X₂₇ denotes the linkage 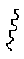 to the other groups in the compound of general formula I.

9. Compound according to any of the preceding claims, wherein group N is the following general group of 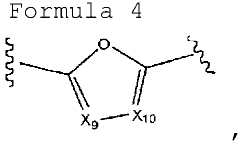 wherein it is preferable that X₉ and X₁₀ are independently of one another selected from C-R where R is in each case independently of one another selected from the group consisting of H, halogen and branched or linear, cyclic or open-chain C₁-C₁₀ alkyl.

10. Compound according to any of the preceding claims, wherein M is the general group of formula 3 and/or N is the general group of formula 6, wherein it is preferable that, in formula 10, A = S or O.

11. Compound according to any of the preceding claims, wherein b = 1 and T2 is the group of formula 10 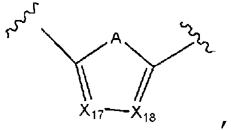 wherein it is preferable that in formula 10 A = S or O, and/or wherein d = 1 and T3 is the group of 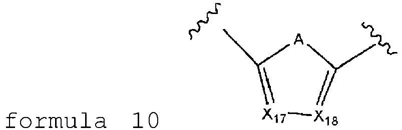 or of formula 11 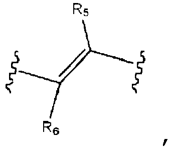 wherein preferably in formula 10 is A = S or O.

12. Compound according to any of the preceding claims, wherein e = 1 and T4 is the group of formula 10 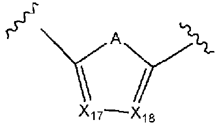 or of 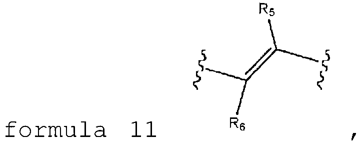 wherein preferably in formula 10 is A = O or S, and/or wherein a = 1 and T1 is the group of formula 10 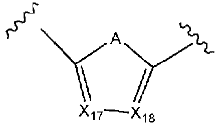 or of formula 11 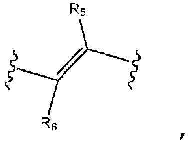 wherein preferably in formula 10 is A = S or O.

13. Compound according to any of the preceding claims, wherein the compound is selected from the group consisting of: 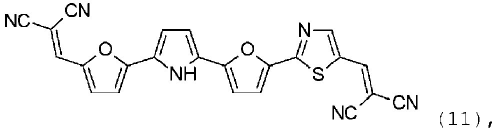 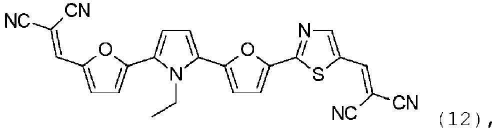 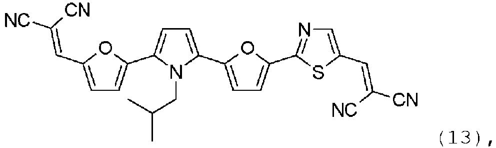 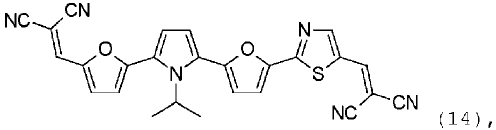 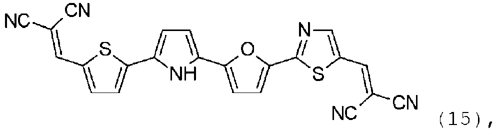 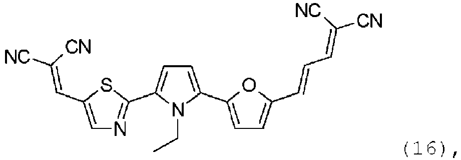 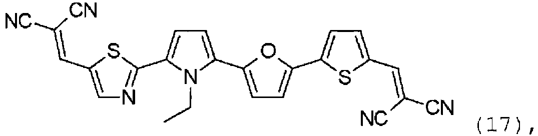 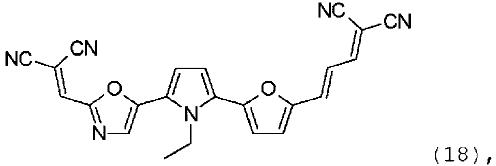 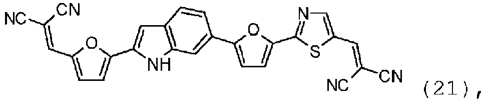 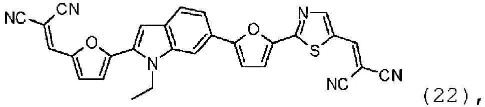 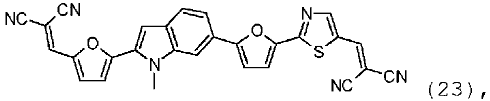 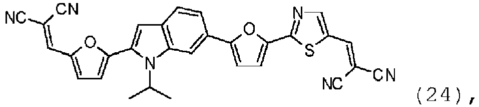 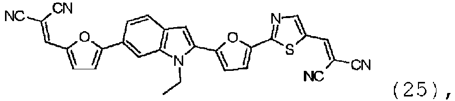 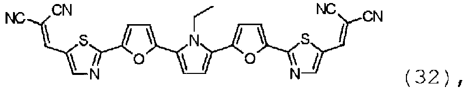 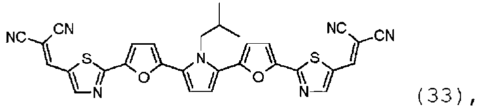 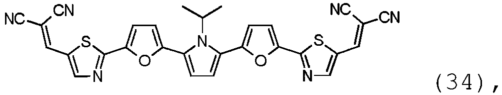 and

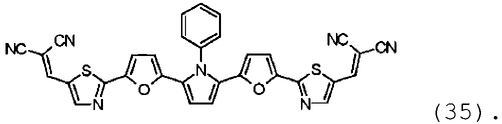

14. Compound according to any of the preceding claims, wherein c = 1, b = 1, and/or e = 1.

15. Use of at least one compound according to any of Claims 1 to 14 in an organic electronic component, preferably in an organic solar cell.

16. Organic electronic component comprising at least one compound according to any of Claims 1 to 14, preferably comprising an electrode (2) and a counter electrode (6) and at least one organic photoactive layer (4) between the electrode and the counter electrode, wherein the organic photoactive layer comprises at least one compound according to any of Claims 1 to 14.

## Revendications

1. Composé de formule générale I
A1-(T1)ₐ-(T2)_{b}-(Z)_{c}-(T3)ₐ-(T4)ₑ-A2 (I)
- les paramètres a, b, d, e valant chaque fois indépendamment les uns des autres 0 ou 1, étant entendu qu'au moins un des paramètres a, b, d, e est égal à 1,
- le paramètre c = 1, 2, ou 3,
- où le groupe général Z est un bloc composé de deux groupes M et N, reliés sous la forme de *-M-N-* ou *-N-M-*, * désignant la liaison aux groupes T1, T2, T3, T4, A1 et A2,
- où les groupes M sont choisis chaque fois indépendamment les uns des autres parmi : 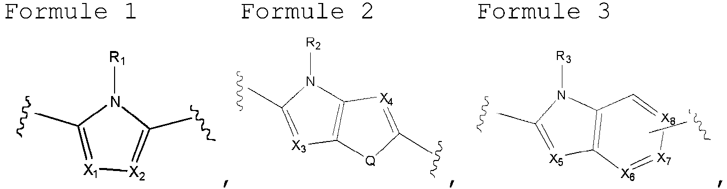
- où les groupes N sont choisis chaque fois indépendamment les uns des autres parmi : 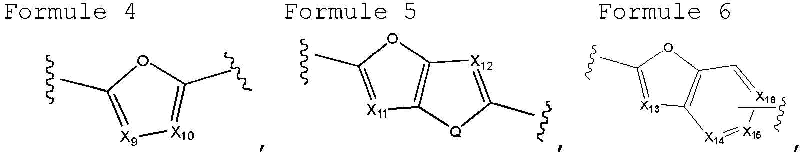
- où M et N sont chaque fois liés de façon telle qu'au moins un atome d'azote du groupe M et un atome d'oxygène du groupe N sont chaque fois reliés entre eux par 2 atomes de carbone, et 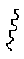 désigne la liaison aux autres groupes dans le composé de formule générale I,
- X₁-X₁₆ étant choisis indépendamment les uns des autres parmi N ou C-R, étant entendu que dans les groupes de formules 3 et 6 un groupe parmi les groupes X₈/X₇ et X ₁₆/X₁₅ désigne chaque fois la liaison 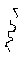 aux autres groupes dans le composé de formule générale I,
- R étant choisi chaque fois indépendamment dans le groupe constitué par H, halogène, alkyle en C₁-C₁₀ ramifié ou à chaîne droite, cyclique ou à chaîne ouverte, des atomes d'hydrogène du groupe alkyle en C₁-C₁₀ pouvant être remplacés et des atomes de carbone du groupe alkyle en C ₁-C₁₀ pouvant être remplacés par des hétéroatomes, alcényle en C₂-C₁₀, O-alkyle, S-alkyle, O-alcényle, S-alcényle, alcynyle, aryle, hétéroaryle, des atomes d'hydrogène dans tous ces groupes pouvant être remplacés, CN, NR₁₀R₁₁, R₁₀ et R₁₁ étant choisis chacun indépendamment l'un de l'autre parmi H, alkyle en C₁-C₁₀ ramifié ou à chaîne droite, cyclique ou à chaîne ouverte, des atomes d'hydrogène du groupe alkyle en C₁-C₁₀ pouvant être remplacés et des atomes de carbone du groupe alkyle en C ₁-C₁₀ pouvant être remplacés par des hétéroatomes,
- R₁ à R₃ étant choisis chacun indépendamment les uns des autres dans le groupe constitué par H, un groupe alkyle en C₁-C₁₀ ramifié ou chaîne droite, cyclique ou à chaîne ouverte, des atomes d'hydrogène du groupe alkyle en C₁-C₁₀ pouvant être remplacés, et des atomes de carbone du groupe alkyle en C₁-C₁₀ pouvant être remplacés par des hétéroatomes, alcényle en C₂-C₁₀ substitué ou non substitué, aryle substitué ou non substitué, hétéroaryle substitué ou non substitué, CN,
- où les groupes A1 et A2 attirant les électrons sont indépendamment les uns des autres des groupes attirant les électrons qui comportent au moins une double liaison C=C,
- où les groupes T1, T2, T3 et T4 sont choisis chacun indépendamment les uns des autres parmi : 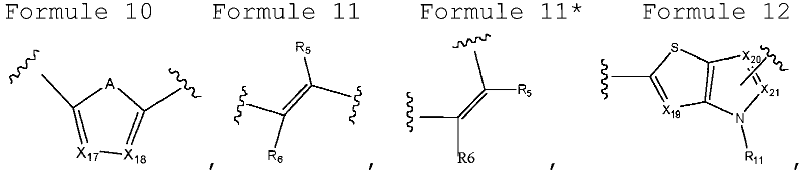 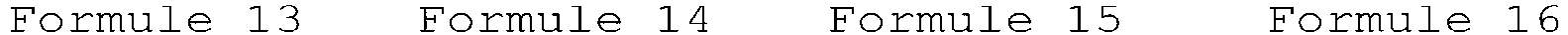 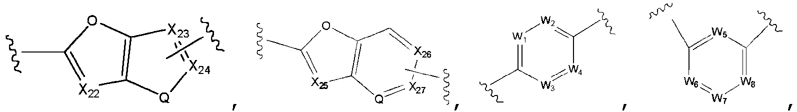
- 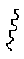 désignant la liaison aux autres groupes dans le composé de formule générale I, étant entendu qu'au moins un des groupes T1 à T4 est de formule 10,
- R₅ et R₆ étant choisis chacun indépendamment l'un de l'autre dans le groupe constitué par H, CN, F, aryle, hétéroaryle, alcényle en C₂-C₁₀, alcynyle, alkyle en C₁-C ₁₀ ramifié ou à chaîne droite, cyclique ou à chaîne ouverte, des atomes d'hydrogène du groupe alkyle en C₁-C ₁₀ pouvant être remplacés, dans le cas ou le substituant R₁₃ est présent dans le composé de formule I, une cyclisation entre R₅ et R₁₃ ou entre R₆ et R₁₃ étant possible, étant entendu que la double liaison de la formule 11 se trouve chaque fois entre R₅ et R₁₃ ou entre R₆ et R₁₃,
- W₁ à W₈ étant choisis chacun indépendamment les uns des autres parmi N, CR, où R est défini comme décrit plus haut,
- X₁₇ et X₁₈ étant choisis indépendamment l'un de l'autre parmi N et C-R, où R est défini comme décrit plus haut, étant entendu qu'au moins X₁₇ ou X₁₈ est N,
- X₁₉ à X₂₇ étant choisis indépendamment l'un de l'autre parmi N et C-R, où R est défini comme décrit plus haut, et étant entendu que dans les groupes de formules 12, 13 et 14, chaque fois un groupe parmi les groupes X₂₀/X₂₁, X ₂₃/X₂₄ et X₂₆/X₂₇ désigne la liaison 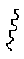 aux autres groupes dans le composé de formule générale I,
- A étant choisi dans le groupe constitué par S, O, NR₉ et Se,
- Q étant choisi dans le groupe constitué par S, O, NR₉ et Se,
- où, pour les groupes A et Q, le substituant R₉ est choisi chaque fois indépendamment parmi H, CN, alkyle en C₁-C₁₀ ramifié ou à chaîne droite, cyclique ou à chaîne ouverte, des atomes d'hydrogène du groupe alkyle en C₁-C ₁₀ pouvant être remplacés, alcényle en C₂-C₁₀, O-alkyle, S-alkyle, O-alcényle, S-alcényle, alcynyle, aryle substitué ou non substitué, hétéroaryle substitué ou non substitué.

2. Composé selon la revendication 1, dans lequel les groupes attirant les électrons A1 et A2 sont choisis indépendamment l'un de l'autre parmi : 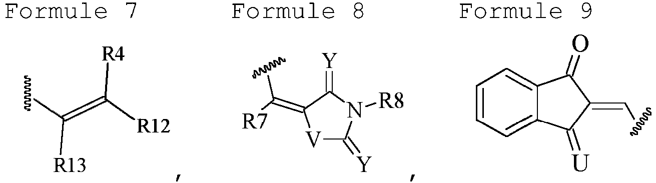 et 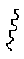 désigne la liaison aux groupes T1 à T4 et Z dans le composé de formule générale I,
- où R₄ et R₁₂ sont choisis chacun indépendamment les uns des autres parmi H, CN et COOR, étant entendu que R₄ et R₁₂ ne sont pas l'un et l'autre H,
- R étant choisi dans le même groupe de composés tel que défini pour R₁ à R₃,
- où R₁₃ est choisi chaque fois indépendamment dans le groupe constitué par H, CN, F, aryle, hétéroaryle, alcényle en C₂-C₁₀, alcynyle, alkyle en C₁-C₁₀ ramifié ou à chaîne droite, cyclique ou à chaîne ouverte, des atomes d'hydrogène du groupe alkyle en C₁-C₁₀ pouvant être remplacés, dans le cas où le substituant R₅ ou R₆ est présent dans le composé de formule I, une cyclisation entre R₅ et R₁₃ ou entre R₆ et R₁₃ étant possible, étant entendu que la double liaison de la formule 11 se trouve entre R₅ et R₁₃ ou entre R₆ et R₁₃, respectivement,
- où V = O, S ; où Y = O, S, C(CN)₂ ; où U = O, S, C(CN)₂,
- où R₇ et R₈ sont choisis chacun indépendamment l'un de l'autre dans le groupe constitué par H, CN, F, aryle, hétéroaryle, alcényle en C₂-C₁₀, alcynyle, alkyle en C₁-C ₁₀ ramifié ou à chaîne droite, cyclique ou à chaîne ouverte, des atomes d'hydrogène du groupe alkyle en C₁-C₁₀ pouvant être remplacés, pour chacun des groupes A1 et A2 respectivement pour chaque double liaison C=C, chaque fois indépendamment l'un de l'autre, aussi bien l'isomère E que l'isomère Z peut être présent.

3. Composé selon la revendication 1 ou 2, dans lequel les groupes attirant les électrons A1 et A2 consistent en le groupe suivant 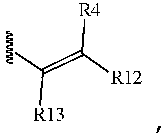 où R₄ et R₁₂ sont de préférence CN.

4. Composé selon l'une quelconque des revendications précédentes où l'un des paramètres a, b, d, e = 0, de préférence où deux des paramètres a, b, d, e = 0, de façon particulièrement préférée où trois des paramètres a, b, d, e = 0, et/ou où au moins l'un des paramètres a, b = 1 et/ou l'un des paramètres d, e = 1, de préférence l'un des paramètres a, b = 0 et l'un des paramètres d, e = 0, de façon particulièrement préférée a et b = 1 et d et e = 1.

5. Composé selon l'une quelconque des revendications précédentes, où c = 1, de formule générale II
A1-(T1)ₐ-(T2)_{b}-Z-(T3)_{d}-(T4)ₑ-A2 (II)
où Z = *-M-N-* ou *-N-M-* et M est un groupe de 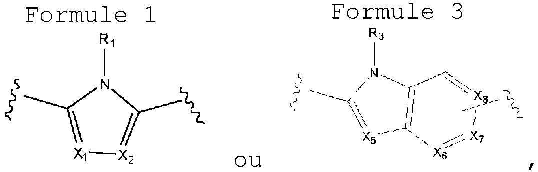 où, de préférence, X₁ et X₂ sont choisis indépendamment l'un de l'autre parmi C-R, R étant choisi chaque fois indépendamment l'un de l'autre dans le groupe constitué par H, halogène, alkyle en C₁-C₁₀ ramifié ou à chaîne droite, cyclique ou à chaîne ouverte.

6. Composé selon l'une quelconque des revendications précédentes où c = 1, ayant la formule générale II
A1-(T1)ₐ-(T2)_{b}-Z-(T3)_{d}-(T4)ₑ-A2 (II),
de préférence T1 ou T2, et T3 ou T4 étant indépendamment les uns des autres de formule 10.

7. Composé selon l'une quelconque des revendications précédentes, dans lequel X₁₇ ou X₁₈ = C-R, où R est choisi chaque fois indépendamment dans un groupe constitué par H, halogène, alkyle en C₁-C₁₀ ramifié ou à chaîne droite, cyclique ou à chaîne ouverte, R₅ et R₆ sont choisis chacun indépendamment l'un de l'autre parmi H, CN, F, aryle, hétéroaryle, alcényle en C₂-C₁₀, alcynyle, alkyle en C₁-C ₁₀ ramifié ou à chaîne droite, cyclique ou à chaîne ouverte, des atomes d'hydrogène du groupe alkyle en C₁-C ₁₀ pouvant être remplacés.

8. Composé selon l'une quelconque des revendications précédentes, où X₁₇ et X₁₈ sont N, et/ou où X₁₉ à X₂₇ sont choisis indépendamment les uns des autres parmi C-R, R étant défini comme décrit plu haut, et étant entendu que dans les groupes de formules 12, 13 et 14 chaque fois un groupe parmi les groupes X₂₀/X₂₁, X₂₃/X₂₄ et X₂₆/X₂₇ désigne la liaison 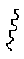 aux autres groupes dans le composé de formule générale I.

9. Composé selon l'une quelconque des revendications précédentes, dans lequel le groupe N est le groupe général suivant de 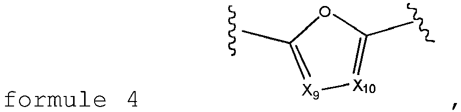 dans laquelle, de préférence, X₉ et X₁₀ sont choisis indépendamment l'un de l'autre parmi C-R, R étant choisi chaque fois indépendamment dans le groupe constitué parmi H, halogène, alkyle en C₁-C₁₀ ramifié ou à chaîne droite, cyclique ou à chaîne ouverte.

10. Composé selon l'une quelconque des revendications précédentes, dans lequel M est le groupe général de formule 3 et/ou N est le groupe général de formule 6, où de préférence, dans la formule 10, A = S ou O.

11. Composé selon l'une quelconque des revendications précédentes, dans lequel b = 1 et T2 est le groupe de 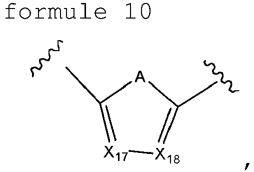 où de préférence dans la formule 10 A = S ou O, et/ou dans lequel d = 1 et T3 est le groupe de 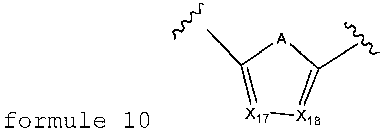 ou de 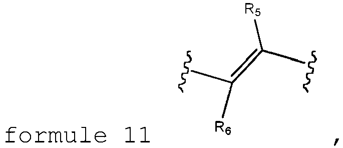 où de préférence dans la formule 10 A = S ou O.

12. Composé selon l'une quelconque des revendications précédentes, dans lequel e = 1 et T4 est le groupe de 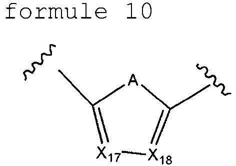 ou de 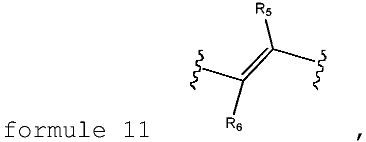 dans lequel de préférence dans la formule 10 A = O ou S, et/ou dans lequel a = 1 et T1 est le groupe de formule 10 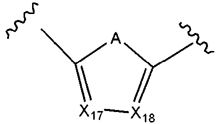 ou de 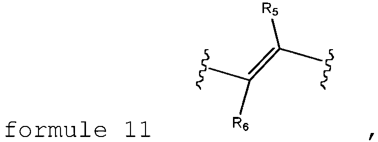 de préférence dans la formule 10 A étant S ou O.

13. Composé selon l'une quelconque des revendications précédentes, dans lequel le composé est choisi dans le groupe constitué par : 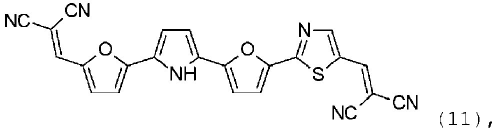 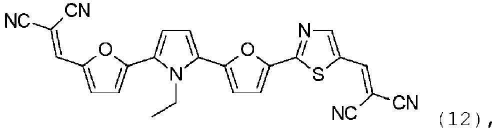 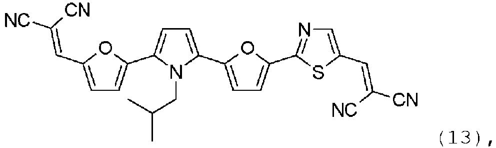 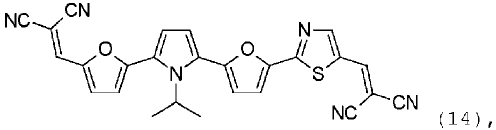 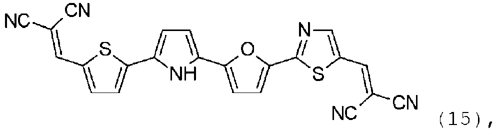 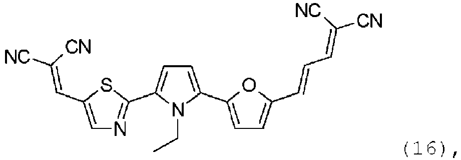 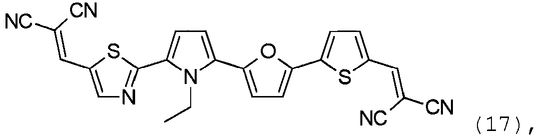 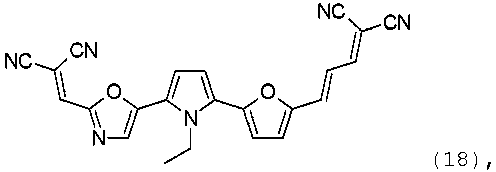 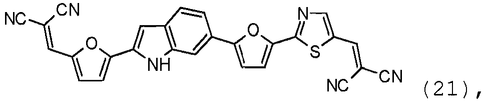 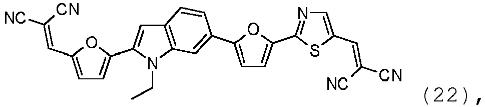 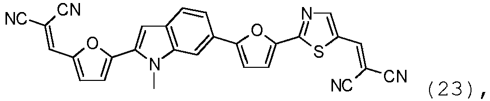 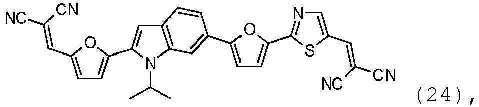 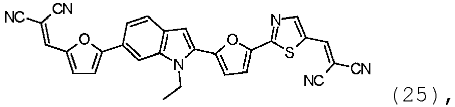 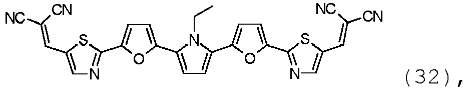 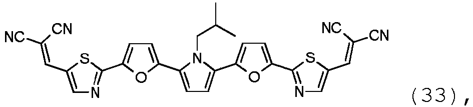 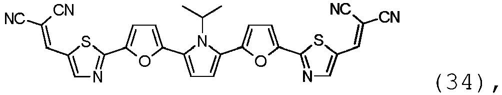 et

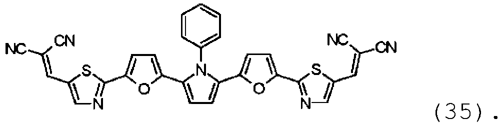

14. Composé selon l'une quelconque des revendications précédentes, dans lequel c = 1, b = 1, et/ou e = 1.

15. Utilisation d'au moins un composé selon l'une quelconque des revendications 1 à 14 dans un composant électronique organique, de préférence dans une cellule solaire organique.

16. Composant électronique organique comprenant au moins un composé selon l'une quelconque des revendications 1 à 14, comprenant de préférence une électrode (2) et une contre-électrode (6) et au moins une couche photoactive organique (4) entre l'électrode et la contre-électrode, la couche photoactive organique comportant au moins un composé selon l'une quelconque des revendications 1 à 14.
